(19) Europäisches Patentamt / European Patent Office / Office européen des brevets

(11) **EP 4 484 960 A2**

(12) **EUROPEAN PATENT APPLICATION**


(43) Date of publication:
**01.01.2025 Bulletin 2025/01**

(21) Application number: **24183943.0**

(22) Date of filing: **24.06.2024**

(51) International Patent Classification (IPC):
***G01N 33/74*** *(2006.01)* ***G01N 33/543*** *(2006.01)*

(52) Cooperative Patent Classification (CPC):
**G01N 33/743; G01N 33/5438;** G01N 2333/575; G01N 2600/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.06.2023 KR 20230081703**



(71) Applicant: **Yonsei University, University-Industry Foundation(UIF).**
**Seoul 03722 (KR)**

(72) Inventors:
- **KIM, Jayoung**
  **03722 Seoul (KR)**
- **LEE, Won-Yong**
  **03722 Seoul (KR)**
- **LEE, Don Hui**
  **03722 Seoul (KR)**

(74) Representative: **Eisenführ Speiser**
**Patentanwälte Rechtsanwälte PartGmbB**
**Postfach 10 60 78**
**28060 Bremen (DE)**


(54) **NANOSCALE MOLECULARLY IMPRINTED POLYMER THIN FILM FOR SMALL MOLECULE DETECTION**


(57) The present invention relates to a nanoscale molecularly imprinted polymer (MIP) thin film for small molecule detection, a method of manufacturing the same, and an electrochemical sensor using the MIP and quantum electrochemical impedance spectroscopy (EIS), and more specifically, to a nanoscale MIP thin film for small molecule detection, wherein a plurality of specific recognition spaces for small molecules of 1000 Da or less are formed in the MIP, a receptor polymer is present at one end of the specific recognition spaces and a redox probe is present in a wire form, and the small molecule is a steroid hormone or a protein, a method of manufacturing the same, and an electrochemical sensor using the MIP and quantum EIS. The sensor according to the present invention is expected to be useful in point-of-care applications because it exhibits a rapid and reversible small molecule detection ability through a simple electrochemical regeneration process without cumbersome washing and solution replacement steps in the manufacturing process, thereby enabling continuous detection.


Fig. 1

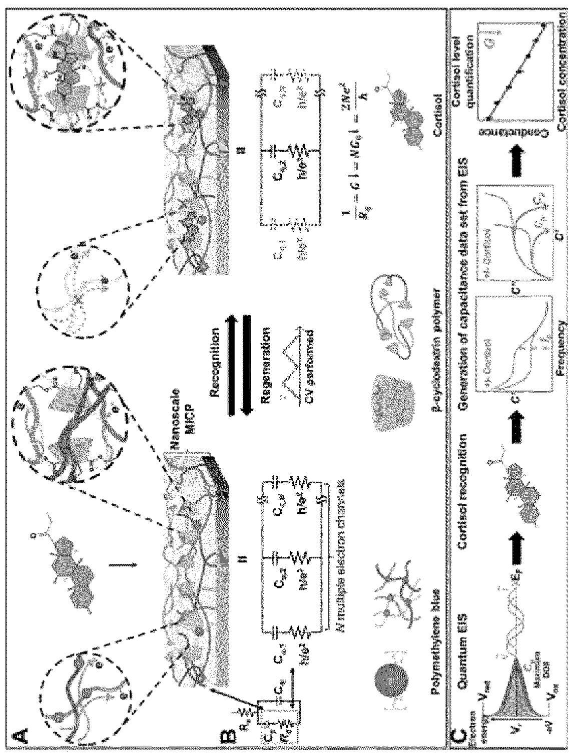


EP 4 484 960 A2

## Description

CROSS-REFERENCE TO RELATED APPLICATION

This application claims priority to and the benefit of Korean Patent Application No. 2023-0081703, filed on June 26, 2023, the disclosure of which is incorporated herein by reference in its entirety.

BACKGROUND

### 1. Field of the Invention

The present invention relates to a nanoscale molecularly imprinted polymer thin film for small molecule detection, a method of manufacturing the same, and an electrochemical sensor using the same.

### 2. Discussion of Related Art

Detection of small molecules such as metabolites, neurotransmitters, and hormones can provide useful biological information for the diagnosis of specific diseases, the prediction of treatment responses, and health condition monitoring. Among various sensing techniques, electrochemical detection has great advantaged for point-of-care (PoC) applications due to its merits, such as easy miniaturization, low cost, and fast responses.

While electroactive small target molecules may be easily detected by direct charge transfer from their own redox reactions on electrode surfaces, electrochemically inactive small molecules can be detected by monitoring the electroactive products released in highly selective enzymatic reactions with target molecules.

However, electrically inactive small molecules, not involving proper enzymatic reactions generating electroactive products, can be detected with affinity-based (bio)sensors, in which small target molecules selectively bind to bioreceptors (e.g., antibodies and aptamers) or biomimetic receptors (e.g., molecularly imprinted polymers).

In general, affinity-based (bio)sensors exhibit extremely high binding affinity, which enables highly selective detection of target molecules but there is difficulty in regenerating receptors, making repetitive and continuous measurements difficult. Moreover, existing affinity-based electrochemical (bio)sensors require either electroactive labels such as labeled secondary antibodies for electrical signal detection or external solutions containing redox probes for electrochemical impedance spectroscopic detection, which are not suitable for PoC application.

Therefore, a great deal of effort has been put into developing "label-free" and "bind-and-read" electrochemical sensing techniques by co-immobilizing (bio)receptors and redox probes on electrode surfaces for decentralized on-site monitoring of clinically important molecules.

In the development of affinity-based (bio)sensors for small molecules, ultrasensitive detection methods are highly desired because of the small molecules' low physiological concentrations ($10^{-12}$ to $10^{-9}$ M level). However, ultrasensitive detection of small molecules faces major challenges because of their inherent imbalance in target-to-receptor size ratio.

Conventional bioreceptors, such as antibodies and aptamers (6 kDa to 180 kDa), are much larger than target small molecules (≤ 1 kDa), so there is little apparent change in their physicochemical properties upon their binding events. In order to achieve highly sensitive and reliable detection, the target-to-receptor size ratio should be higher than 0.3.

Thus, some efforts have been made to reduce the size of biological receptors by utilizing nanobodies and truncated aptamers. However, the above problem has not been completely resolved because the resulting target-to-receptor size ratio is still smaller than 0.3 [Non-patent Documents 1 and 2].

Reducing the molecularly imprinted polymer (MIP) thickness could increase the target-to-receptor size ratio, which results in pronounced changes in their physicochemical properties upon their binding events, thus leading to the highly sensitive detection of small target molecules. Conventionally, electrochemical detections in MIPs are mostly based on the measurement of charge transfer resistance ($R_{ct}$) at the MIP interface (interface with solution of entire MIP including cavity) upon the binding of target molecules using an external redox probe in the measurement solution via the electrochemical impedance spectroscopy (EIS) technique (referred to as $R_{ct}$ EIS) [Non-patent Document 3]. However, because the $R_{ct}$ of the thin film itself is very low, the binding of small target molecules may not induce noticeable changes in $R_{ct}$, so $R_{ct}$ EIS may not be suitable for the detection of small molecules in nanometer-sized thin MIP films.

Therefore, an alternative that enables highly sensitive signaling while resolving the imbalance in the target-to-receptor size ratio is needed

[Related Art Documents]
[Non-patent Documents]

(Non-patent Document 1) Kim, Y.; Yang, J.; Hur, H.; Oh, S.; Lee, H. H. Highly sensitive colorimetric assay of cortisol using cortisol antibody and aptamer sandwich assay. Biosensors 2021, 11 (5), 163.

(Non-patent Document 2) Ding, L.; Wang, Z.; Zhong, P.; Jiang, H.; Zhao, Z.; Zhang, Y.; Ren, Z.; Ding, Y. Structural insights into the mechanism of single domain VHH antibody binding to cortisol. FEBS letters 2019, 593 (11), 1248-1256.

(Non-patent Document 3) Apodaca, D. C.; Pernites, R. B.; Ponnapati, R.; Del Mundo, F. R.; Advincula, R. C. Electropolymerized molecularly imprinted polymer film: EIS sensing of bisphenol A. Macromolecules 2011, 44 (17), 6669-6682.

SUMMARY OF THE INVENTION

Therefore, the present inventors have made research efforts to solve the above problems, and as a result, they manufactured a nano-sized molecularly imprinted polymer (MIP) that does not require the cumbersome process of replacing the solution from a target binding solution (biological sample) to a redox measurement solution and an electrode cleaning step and that allows on-site electrochemical regeneration, overcoming the limitations of existing affinity-based (bio)sensors, such as single measurement or the need for cumbersome and complicated regeneration processes, and enabling repeated/continuous measurement. Consequently, the present inventors completed the present invention by developing a novel electrochemical sensing technique for ultrasensitive detection of small molecules.

Therefore, one object of the present invention is to provide a small molecule-selective MIP thin film manufactured by electrochemically copolymerizing β-cyclodextrin (β-CD) as receptor and methylene blue (MB) as a redox probe, and a method of manufacturing the same.

In addition, another object of the present invention is to provide an electrochemical biosensor based on the MIP and an electrochemical sensor for detecting small molecules using the same.

Hereinafter, the present invention will be described in more detail.

The present invention relates to a nanoscale MIP thin film for small molecule detection, a method of manufacturing the same, and an electrochemical sensor using the same.

As one embodiment, the present invention includes a nanoscale MIP thin film for small molecules, in which a plurality of specific recognition spaces for small molecules of 1000 Da or less are formed, wherein a receptor (e.g., β-cyclodextrin) polymer is present at one end of the specific recognition spaces, a redox probe is present in a wire form, and the small molecules are a steroid hormone or a protein.

In the present invention, the term "small molecule" refers to a steroid hormone with a molecular weight of 1000 Da or less (1 to 1000 Da), and the steroid hormones include cortisol (362.46 g/mol), melatonin (232.278 g/mol), and the like. In the present invention, a small molecule is used as a target analyte (detection).

Cortisol is a representative non-electroactive small molecule (molecular weight: 362.46 Da), and it was selected as a target analyte of the present invention because it is present at a low physiological concentration (serum 83 to $690\times10^{-9}$ M, sweat 0.66 to $397.3\times10^{-9}$ M, saliva 2.2 to $27.3\times10^{-9}$ M) along with steroids having a similar chemical structure.

The term "molecularly imprinted polymer (MIP)" used in the present specification refers to a polymer that is formed by synthesizing a polymer using a suitable template (a small molecule such as cortisol in the present invention) and removing the template so that the polymer may memorize the shape of the template and include the same space as the template. MIPs have been applied in various fields such as detection, recognition, removal, extraction, delivery, and analysis of specific substances due to their high selectivity and stability.

The technique for obtaining the MIPs, that is, the molecular imprinting technique, is capable of forming a three-dimensional polymer structure by adding any template material to a solution in which monomers are dissolved, and after removing the template material therefrom, cavities that may selectively bind to the template material are formed.

The MIP of the present invention is prepared by electrochemically copolymerizing small molecules, a receptor, and a redox probe, and then removing the small molecules. Preferably, electrochemical polymerization may be performed with the small molecules, the receptor, and the redox probe at a molarity(M) ratio of 1:0.5 to 1.5:5 to 15. The above ratio is a ratio for optimizing the performance of a sensor, and when the ratio is changed, a change in detection performance through the synthesized polymer thin film occurs.

The electrochemical copolymerization may be performed by 7 to 12 cycles of cyclic voltammetry (CV) scans between -2.0 and 2.2 V at a scan rate of 50 to 200 mV/s.

In the present invention, the term "cavity" refers to a space that may selectively bind to a template material within an MIP and means "specific molecular recognition space." To create a specific molecular recognition space, extraction of a target molecule (small molecule) from an MIP thin film may be achieved by a number of methods, including washing, sonication, and electrochemical peroxidation. Preferably, extraction of the small molecules from the polymer film includes applying electrochemical peroxidation to the MIP thin film.

"Electrochemical peroxidation" oxidizes functional groups of a receptor through CV to weaken the hydrogen

bond between these functional groups and the small molecules, allowing the small molecules to be separated from the MIP.

In the present invention, the term "receptor" refers to a substance or polymer itself that is fixed to a surface of an electrode and selectively binds to a target. Monomers for forming the polymer may include β-cyclodextrin, pyrrole, ortho-phenylenediamine. The selective binding means that the (hydrogen) bond between the receptor and the small molecules in the MIP becomes weaker under oxidizing conditions.

In the present invention, the term "redox probe" may be a molecule or a component part of the MIP, which may undergo an oxidation or reduction reaction. In addition, it is preferable in the present invention to use Prussian Blue, Ferrocene, or methylene blue as a redox probe that may generate constant signals for repetitive electrochemical oxidation/reduction reactions.

In one embodiment of the present invention, "β-cyclodextrin polymer" in an MIP serves as a recognition element for a target analyte, and a co-bound polymethylene blue (PMB) serves as a "wire" in which a polymer chain connects a redox site with a resonance charge to an electrode, and when a target molecule (small molecule) binds to the cavity within the MIP, electron transfer through the adjacent PMB is disrupted, lowering the conductance of the PMB wire and reducing resonant quantum conductance.

In the present invention, the term "nanoscale MIP thin film" refers to a thin film having a thickness of 5 nm or less, preferably 0.1 to 5 nm. The thickness of an MIP film is an important factor that affects not only the reproducibility of the sensor but also the recognition ability of the film. The film thickness may be controlled by adjusting the concentration of monomers and the number of polymerization cycles.

In addition, the present invention includes method of manufacturing a nanoscale MIP thin film for small molecules, including:

forming an MIP thin film for small molecules on an electrode of an electrochemical sensing device; and
removing the small molecules.

All of the above-described content in relation to the MIP thin film may be directly applied or adapted to the method of manufacturing the MIP thin film.

The "quantum electrochemical detection device" used in the present specification may be a device measuring resonant quantum conductance using quantum electrochemical impedance spectroscopy (EIS).

The term "quantum electrochemical impedance spectroscopy (EIS)" used in the present specification refers to a method of measuring the interference with electron transfer through the oxidation/reduction material contained in an MIP thin film when a target material is bound to the thin film as conductance. While conventional EIS reads the charge transfer resistance of a thin film shown in the Nyquist plot, quantum EIS reads the resonant quantum conductance, which is a quantum characteristic of nanoscale thin films.

The electrode may be pretreated by ultrasonic treatment in ethanol and deionized water and then performing an electrochemical potential sweep.

The small molecules may be removed from an MIP through electrochemical peroxidation in which functional groups of a receptor are oxidized through CV to weaken the hydrogen bond between the functional groups and the small molecules. Specifically, small molecules may be removed by performing 7 to 12 cycles of CV scans between 0 and 0.9 V at a scan rate of 50 to 150 mV/s.

In addition, the present invention includes a method of detecting small molecules, including:

Step 1 of bringing a biological sample into contact with a quantum electrochemical detection device including an MIP thin film;
Step 2 of applying voltage to the quantum electrochemical detection device; and
Step 3 of monitoring changes in resonant quantum conductance from the quantum electrochemical detection device when a targeted small molecule binds to a specific recognition space inside the MIP thin film.

Step 1 is a step of bringing a biological sample into contact with a manufactured quantum electrochemical detection device, and it includes noncovalently bonding a targeted small molecule present in the biological sample to a cavity generated in the MIP thin film through hydrogen bonding and a size effect.

The biological sample is selected from the group consisting of plasma, serum, saliva, urine, mucus, and tears of a human or animal.

The contact means bringing a biological sample into contact with a surface of the manufactured quantum electrochemical detection device for 2 to 20 minutes.

Step 2 is a step of reading the electrical characteristics that change when a small molecule binds to the MIP thin film using a quantum electrochemical detection device, and it includes applying a direct current (DC) voltage (-0.1 V to -0.3 V), which is an intermediate redox voltage of a redox probe immobilized in the MIP thin film, to the detection

device as and simultaneously applying an alternating current (AC) voltage of 5 to 20 mV while changing the frequency (0.01 Hz to 10 kHz).

Step 3 is a step of converting the data obtained when the voltage is applied to resonant quantum conductance, wherein the data may be converted into data on capacitance using Mathematical Formulas 6 and 7 described in the Examples, and the resonant quantum conductance may be calculated through the data on capacitance (see Mathematical Formula 21).

It is possible to calculate the concentration of cortisol from the resonant quantum conductance through quantum electrochemical impedance measurement.

In one embodiment, the present invention may further include a step of calculating the concentration of small molecules in a biomaterial using the measured resonant quantum conductance and a previously prepared calibration curve for each concentration of the small molecules.

Specifically, to detect cortisol as a small molecule, calculation was performed using the calibration curve in the attached drawing FIG. 13.

The term "resonant quantum conductance" used in the present specification refers to the conductance when current charging/discharging occurs at the same rate in the oxidized/reduced portions of the nano-sized thin film (when density of state (DOS) reaches a maximum value).

The present invention also includes an electrochemical biosensor for small molecule detection, including the MIP thin film for small molecules.

In the present invention, the term "electrochemical sensor" may be understood as a device configured to detect the presence and/or measure the concentration of an analyte through an electrochemical oxidation and/or reduction reaction.

In one embodiment, an electrochemical biosensor for small molecule detection including the MIP thin film for small molecules utilizes changes in the quantum properties of the thin film. The changes in the quantum properties of the thin film are caused by a local change that occurs when a small molecule binds to the nanoscale thin film. As one embodiment, in an Example of the present invention, FIG. 6 shows changes in the electrochemical capacitance ($C_\mu$) and RC time constant ($f_p$), and values for these changes may be calculated as resonant quantum conductance through Mathematical Formula 21 described in the Examples below. Since polymethylene blue is immobilized in the MIP thin film as a redox probe, when a voltage is applied, a current flowing through the probe follows the electron hopping method, and thus when a target material is attached to the surrounding area, the current flow is interrupted and reduced, resulting in changes in the resonant quantum conductance. An electrochemical biosensor for small molecule detection including the MIP thin film for small molecules utilizes this quantum property change principle. The biosensor detects small molecules using the changes in the redox current flowing through the redox probe in the thin film when a voltage is applied to measure the changes in quantum properties. These quantum property changes occur when a small molecular binds to the cavity of the MIP thin film, and among the quantum properties, the greatest change occurs in the resonant quantum conductance, allowing small molecule detection based on this principle. The thickness of the MIP thin film of the biosensor is 5 nm or less (0.1 nm ~ 5 nm).

In addition, in one embodiment where the small molecule is cortisol, the MIP thin film of the biosensor may be formed by copolymerization of a receptor polymer (e.g., β-cyclodextrin polymer) and a redox probe (e.g., polymethylene blue), and it may preferably be a single layer. In addition, the MIP thin film is preferably formed by electrochemical polymerization of cortisol, β-cyclodextrin, and methylene blue at a molarity(M) ratio of 1:0.5 to 1.5:5 to 15. When the molar concentration of each material is outside the above range or the thickness becomes thicker, changes in electrochemical properties depending on the presence or absence of a bond between the MIP thin film and a target molecule may be detected, but there is a disadvantage in that accuracy is significantly reduced. All of the above-described content regarding the MIP thin film may be directly applied or adapted to the biosensor.

Three electrodes (three terminal electrodes) may be used in the biosensor. In addition, the biosensor may have a structure in which a working electrode, a reference electrode, and a counter electrode are formed in the same solution. In the biosensor, a change in resonant quantum conductance occurs depending on the presence or absence of a bond between an MIP thin film and a small molecule. In other words, the biosensor does not simply sense differences in electrical conductance or resistance, but it detects a small molecule using the changes in resonant quantum conductance due to the changes in the current flowing through a redox material in the MIP thin film when the frequency of the AC voltage is changed while both DC and AC voltages are applied at the same time for resonant quantum conductance.

Since the sensor according to the present invention exhibits a detection limit of $1.0\times10^{-13}$ to $1.0\times10^{-6}$, which is much lower than that of the existing electrochemical sensors, ultrasensitive on-site detection is possible.

The sensor according to the present invention may be commercialized so that the small molecule concentration may be displayed on the display when the sensor is immersed in a sample (in the same manner as a blood sugar sensing device).

While measurements based on EIS and differential pulse voltammetry (DPV) are not sensitive enough to generate a distinct signal change induced by a local change upon a target analyte binding event because they basically

rely on the measurement of the redox reaction governed by electron transfer throughout the entire film, the measurement of resonant quantum conductance reflects a local change in the charge density of the redox probe wire in the nanoscale MIP thin films and thus offers superior sensing performance in terms of sensitivity, detection limit, and dynamic range compared to other conventional methods.

In addition, the present invention provides a method of manufacturing a biosensor for small molecule detection using changes in resonant quantum conductance, including: forming an MIP thin film on a working electrode (Step A); forming a nanoscale thin film of 5 nm or less by performing electrochemical polymerization of small molecules, β-cyclodextrin, and a redox probe at a molarity(M) ratio of 1:0.5 to 1.5:5 to 15 on the working electrode and then electrochemically separating the small molecules from the electropolymerized thin film (Step B).

In Step A, electrochemical copolymerization is possible by reducing the functional groups of the receptor through CV and forming hydrogen bonds between the small molecules and the functional groups of the receptor. Specifically, electrochemical copolymerization is performed by 7 to 12 cycles of CV scans between -2.0 and 2.2 V at a scan rate of 50 to 200 mV/s.

Step B is carried out through an electrochemical change in the structure of the MIP thin film. In other words, the functional groups of the receptor are oxidized through CV to weaken the hydrogen bond between the small molecules and the functional groups of the receptor, allowing the small molecules to be extracted. The electrochemical MIP thin film structural change in Step B is performed by 7 to 12 cycles of CV scans between 0.0 and 0.9 V at a scan rate of 50 to 200 mV/s.

In addition, the present invention provides a method of sensing a small molecule using a biosensor for small molecule detection including a working electrode, a reference electrode, and a counter electrode provided in one cell and spaced apart from each other; an electrolyte solution in which the three electrodes are immersed; an MIP thin film electrochemically copolymerized with the working electrode and binding to a small molecule, the MIP film having a thickness of 5 nm or less and including a redox probe, and formed of the MIP thin film for small molecule detection, the method including: bringing a biological sample into contact with the MIP thin film; and measuring the quantum properties of the thin film before and after contact between the quantum electrochemical detection device and the sample and confirming changes in resonant quantum conductance.

In one embodiment, the sensing method is performed by detecting a change in resonant quantum conductance depending on the presence or absence of a bond between an MIP thin film and a small molecule.

In other words, the biosensor does not simply sense differences in electrical conductance or resistance, but it detects a small molecule using the changes in resonant quantum conductance due to the changes in the current flowing through a redox material in the MIP thin film when the frequency of the AC voltage is changed while both DC and AC voltages are applied at the same time for resonant quantum conductance.

## BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

FIG. 1 shows an ultrasensitive cortisol sensor using quantum electrochemical impedance spectroscopy (EIS) with a molecularly imprinted copolymer (MICP) [(A) Schematic diagram of the redox immobilized MICP, including a β-cyclodextrin polymer (CDP) and polymethylene blue (PMB) for reversible cortisol recognition; (B) Equivalent circuit of the MICP with a PMB electron channel modeled as a quantum conductor coupled to a quantum capacitor; and (C) Schematic diagram of a proposed approach for ultrasensitive cortisol detection by using quantum EIS as a capacitive signal transduction method that can measure the changes in quantum properties in nanoscale MICPs];

FIG. 2. shows the electrochemical preparation and characterization of the MICP [(A) CV curves (1st to 10th cycle) for electrochemical copolymerization of β-cyclodextrin (β-CD) and methylene blue (MB) on a glassy carbon electrode (GCE); (B) The chemical reaction for MB electropolymerization; (C) Chemical reactions for the β-CD electropolymerization; (D) CV curves of bare GCE, CDP only, PMB only, and MICP-modified GCE; (E) Consecutive CV scans at the MICP electrode. $V_{read}$ (($PMB_{ox}$ + $PMB_{red}$)/2) is a formal potential of PMB; and (F) An atomic force microscopy (AFM) image of the MICP-modified glassy carbon plate with a height distribution histogram.];

FIG. 3 shows field emission-scanning electron microscope (FE-SEM) images of the modified GCE [(A) Electropolymerized CDP. Inset, bare GCE plate; (B) Electropolymerized PMB; Electrochemically copolymerized MICP with β-CD, MB, and cortisol before (C) and after (D) electrochemical extraction; and an electrochemically copolymerized non-imprinted copolymer (NICP) with β-CD and PMB without cortisol before (E) and after (F) electrochemical extraction; Scale bar, 500 nm.];

FIG. 4 shows the differential pulse voltammetry (DPV) curves of an MICP and NICP before and after electrochemical extraction [performed between 0 V and -0.4 V, with a pulse amplitude of 50 mV to measure the reduction peak

current of PMB];

FIG. 5 shows the UV-vis spectra of the CDP only, PMB only, and MICP-modified indium tin oxide glass;

FIG. 6 shows electrochemical cortisol detection using quantum EIS [Impedance Nyquist plots (A), a calibration plot of relative $Z_{im}$ with a linear fit (B), capacitive Nyquist plots (C), real capacitance Bode plots (D), imaginary capacitance Bode plots (E), and a calibration plot of relative $C_{\mu}$ with a linear fit (F) obtained at various cortisol concentrations. Solution resistance ($R_s$) obtained from (A), electrochemical capacitance ($C_{\mu}$) from (B) & (C), and peak frequency ($f_p$) from (D) were used for resonant quantum conductance ($G$) calculation. (G) A calibration plot of relative $G$ with a linear fit. (H) Electron transfer rate ($k$) response as a function of cortisol concentration. (I) Relative responses of $C_{\mu}$, $k$, and $G$ as a function of cortisol concentration. All error bars represent standard deviations of independent measurements (n = 4).];

FIG. 7 shows the *G* response as a function of cortisol concentration. Inset, the plot of *G* versus log [cortisol concentration];

FIG. 8 shows impedance Nyquist plots obtained from a bare GCE and nanoscale MICP-modified GCE (electrochemically copolymerized for 10 cycles) in the presence of 5.0 mM $K_3Fe(CN)_6/K_4Fe(CN)_6$ in 1x PBS (pH 7.4);

FIG. 9 shows electrochemical cortisol detection based on charge transfer resistance ($R_{ct}$) EIS [(A) Impedance Nyquist plots for thick MICP modified GCE (electrochemically copolymerized for 40 cycles) obtained at various cortisol concentrations with 5.0 mM $K_3Fe(CN)_6/K_4Fe(CN)_6$; and (B) Relative $R_{ct}$ as a function of cortisol concentration with a linear fit. Error bars represent the standard deviations of independent measurements (n = 3).];

FIG. 10 shows electrochemical cortisol detection using differential pulse voltammetry (DPV) [(A) DPV graphs of the MICP-based cortisol biosensor obtained at various cortisol concentrations; (B) Relative peak current (*I*) as a function of cortisol concentration with a linear fit; and all error bars represent standard deviations of independent measurements (n = 3)];

FIG. 11 shows evaluation of the MICP as an artificial receptor [(A) Resonant quantum conductance (*G*) response versus extraction cycles after incubation in 100 nM cortisol with initial conductance; (B) The proposed chemical reaction for electrochemical regeneration of the MICP; (C) Reversible detection of 100 nM cortisol using the MICP sensor; (D) Relative *G* response versus binding time at various concentrations of cortisol; (E) The observed rate constant of the binding reaction ($k_{obs}$) as a function of cortisol concentration; (F) Experimental adsorption isotherm with fitted corresponding adsorption isotherms; (G) Selectivity test of an MICP and non-imprinted copolymer (NICP) in the presence of cortisol and structural analogs at 1 nM, respectively; (H) Long-term stability of the MICP cortisol sensors; and all error bars represent the standard deviations of independent measurements (n = 3).];

FIG. 12 shows selectivity of the MICP and NICP in the presence of cortisol and structural analogs at 100 nM (A), and 1 pM (B), respectively [All error bars represent the standard deviations of independent measurements (n = 3)]; and

FIG. 13 shows the results of validating cortisol sensing performance in real human saliva [(A) The real capacitance Bode plots obtained at various concentrations of cortisol. Inset, the enlarged version at 0.08224 Hz; (B) The imaginary capacitance Bode plots. Inset, the enlarged version at 1.35542 Hz; (C) The calibration plot of relative *G* response for different cortisol concentrations with a linear fit; (D) Schematic diagram of the process of monitoring the cortisol level in human saliva which follows a circadian rhythm by the MICP-based cortisol sensor, along with liquid chromatography-mass spectrometry/mass spectrometry (LC-MS/MS) validation; (E) Repetitive detection of the saliva sample collected in the morning and evening through electrochemical regeneration steps; (F) Salivary cortisol detection throughout the day using the MICP-based cortisol sensor and LC-MS/MS; and all error bars represent the standard deviations of independent measurements (n = 3).].

DETAILED DESCRIPTION OF EXEMPLARY EMBODIMENTS

Hereinafter, the present invention will be described in more detail through examples according to the present invention, but the scope of the present invention is not limited to the examples presented below.

**[Examples]**

**< Chemicals and Instrumentations** >

The following chemicals were used without separate purification steps: 1.00 μm alumina powder (40-10079, Buehler), 0.05 μm gamma alumina powder (90-187050, ALLIED), ethanol (94.5 %, DAEJUNG), β-cyclodextrin hydrate (99%, Sigma Aldrich), methylene blue hydrate (>_ 97 %, Sigma Aldrich), hydrocortisone (>_ 98 %, Sigma Aldrich), phosphate buffered saline (10×, Sigma Aldrich), sulfuric acid (95.0 - 98.0 %, Sigma Aldrich), prednisolone (>_ 99 %, Sigma Aldrich), progesterone (>_ 99 %, Sigma Aldrich), calcium chloride (anhydrous, ≥ 97 %, Sigma Aldrich), potassium hexacyanoferrate (II) trihydrate (≥ 99.95 %, Sigma Aldrich), potassium hexacyanoferrate (III) (≥ 99.0 %, Sigma Aldrich), sodium bicarbonate (≥ 99.7 %, Sigma Aldrich), sodium phosphate dibasic (>_ 99 %, Sigma Aldrich), potassium chloride

(>_ 99 %, Sigma Aldrich), urea (>_ 99 %, Sigma Aldrich), sodium hydroxide (≥ 98 %, Sigma Aldrich), a cortisol solution (1 mg/mL in methanol, Sigma Aldrich), sodium chloride (≥ 99 %, Sigma Aldrich), a cortisol-D4 (9, 11, 12, 12-D4) solution (100 μg/ml in methanol, Sigma Aldrich), pure ethyl alcohol (200 proof, Sigma Aldrich), and methanol (≥ 99 %, Sigma Aldrich); Deionized (DI) water for all solutions was purified using a Milli-Q water purification system (Millipore, Bedford, MA, USA).

Unless otherwise stated, all solutions were dissolved in a 1× PBS (pH 7.4) solution.

All electrochemical experiments were performed using a CS350 potentiostat (Corrtest Instruments Co., Ltd.). A three-electrode electrochemical cell was composed of a GCE (MF-2012, BAS, Inc., 3 mm in diameter) as a working electrode, a platinum counter electrode (002222, ALS Co., Ltd), and an Ag/AgCl (3 M NaCl) reference electrode (MF-2052, BAS, Inc.). The FE-SEM images were obtained using JEOL-7800F (JEOL, Ltd.). The AFM images were obtained in a tapping mode using NX-10 (Park Systems). UV-vis spectra were acquired using V770 (JASCO, Ltd.). Liquid chromatography-mass spectrometry/mass spectrometry (LC-MS/MS) analysis of cortisol in human saliva samples was performed using Ultimate 3000 RS-Q-Exactive Orbitrap Plus (ThermoFisher Scientific).

Hereinafter, the MIP film for cortisol is referred to as "MICP."

**Reference Example 1: Theoretical description of quantum EIS**

In order to charge/discharge electrochemical redox states of "PMB wire" spanning the space between the electrode and redox site, the conduction of a specific potential gradient (*dV*) induces a resonant exchange of electrons, where V is potential. The chemical potential ($\mu$) of this wire is expressed as Mathematical Formula 1:

[Mathematical Formula 1]

$$dV = -\frac{d\mu}{e}$$

where e is the elementary charge. For an ideal quantum mechanical electrical channel, the current gradient (*di*) is related to $\mu$ through Mathematical Formula 2:

[Mathematical Formula 2]

$$di = -d\mu \frac{ev}{L} \frac{\delta N}{\delta \mu}$$

where v is the charge velocity along the channel length *L*, *N* is the number of electrons, and $\delta N/\delta \mu$ is the density of state (DOS) which is proportional to electrochemical capacitance ($C_\mu$) [Bueno, P. R.; Fernandes, F. C. B.; Davis, J. J. Quantum capacitance as a reagentless molecular sensing element. Nanoscale 2017, 9 (40), 15362-15370.; Bueno, P. R. Nanoscale Electrochemistry of Molecular Contacts; Springer, 2018.]. At nanoscale, the wire's electronic properties show a dependency on the wire DOS through Mathematical Formula 3:

[Mathematical Formula 3]

$$\frac{\delta N}{\delta \mu} = \frac{2L}{hv}$$

where *h* is the Planch constant. Considering the above Mathematical Formulas 1, 2, and 3, the corresponding electric current gradient can be expressed as Mathematical Formula 4:

[Mathematical Formula 4]

$$di = N\frac{2e^2}{h}dV$$

Finally, current (i) is related to resonant quantum conductance (G) through Mathematical Formula 5:

[Mathematical Formula 5]

$$i = GV \ \text{(where, } G = NG_0 = \frac{2Ne^2}{h}\text{ )}, G_0 = \frac{2e^2}{h}$$

where $G_0$ is quantized conductance. A target binding event disrupts electron transfer through the "PMB wire," thereby decreasing charge velocity and current. The changes in charge velocity and current can be measured as $C_\mu$, and $G$, respectively.

**Reference Example 2: Conversion of impedance data to capacitance data**

Capacitance data was obtained from quantum EIS experiments using Mathematical Formulas 6 and 7 below [Garrote, B. L.; Santos, A.; Bueno, P. R. Label-free capacitive assaying of biomarkers for molecular diagnostics. Nature Protocols 2020, 15 (12), 3879-3893.].

Using Mathematical Formulas 6 and 7 below, *C'* and *C"* can be calculated using $Z_{im}$, $Z_{re}$, $|Z|^2$, and $\omega$, which are data values obtained after measuring EIS, respectively.

[Mathematical Formula 6]

$$C'(\omega) = Z_{im}/\omega\ |Z|^2$$

[Mathematical Formula 7]

$$C''(\omega) = Z_{re}/\omega\ |Z|^2$$

where $\omega$ is the angular frequency ($\omega = 2\pi f$).

**Reference Example 3: Estimation of MICP thickness using surface coverage**

The thickness of each polymer film (PMB and CDP) on the GCE can be estimated from the corresponding CV peaks generated during the electrochemical copolymerization process. The surface coverage ($\Gamma$) of PMB and CDP can be expressed respectively as Mathematical Formula 8 [Marinho, M. I. C.; Cabral, M. F.; Mazo, L. H. Is the poly (methylene blue)-modified glassy carbon electrode an adequate electrode for the simple detection of thiols and amino acid-based molecules? Journal of Electroanalytical Chemistry 2012, 685, 8-14.].

[Mathematical Formula 8]

$$\Gamma = \frac{Q}{nFA}$$

where Q is equivalent to the total amount of charge involved in the polymerization, n is the number of electrons for the electrochemical reaction, which is 2 for PMB and 4 for CDP, *F* is Faraday's constant (96485.34 C/mol) and *A* is the electrode surface area (0.070686 $cm^2$). The Q value was obtained by integrating the current of PMB and CDP at a scan rate of 100 mV/s over the potential range (+0.1 to 0.8 V for PMB, +0.9 to 1.75 V for CDP) during their polymerization process. The film thickness (*d*) of PMB and the CDP was then calculated from $\Gamma$ by Mathematical Formula 9.

[Mathematical Formula 9]

$$d = v \times \Gamma$$

where *v* is the molecular volume, 400 cm3/mol for PMB and 317 $cm^3$/mol for CDP. [Tan, L.; Xie, Q.; Yao, S. Electrochemical and spectroelectrochemical studies on pyridoxine hydrochloride using a poly (methylene blue) modified electrode. Electroanalysis: An International Journal Devoted to Fundamental and Practical Aspects of Electroanalysis 2004, 16 (19), 1592-1597.; Sandilya, A. A.; Natarajan, U.; Priya, M. H. Molecular view into the cyclodextrin cavity: structure and hydration. ACS Omega 2020, 5 (40), 25655-25667]. The film thickness (*d*) of the PMB and CDP was determined to be 1.97 and 3.11 nm, respectively.

**Reference Example 4: Calculation of MICP binding constants using binding kinetics**

The plots of relative G response versus binding time at various cortisol concentrations were fitted by using "binding kinetics (one ligand concentration)" to estimate $k_{off}$ and $k_{on}$ using Mathematical Formula 10 below [Zeilinger, M.; Pichler, F.; Nics, L.; Wadsak, W.; Spreitzer, H.; Hacker, M.; Mitterhauser, M. New approaches for the reliable in vitro assessment of binding affinity based on high-resolution real-time data acquisition of radioligand-receptor binding kinetics. EJNMMI Research 2017, 7 (1), 1-13.; Jarmoskaite, I.; AlSadhan, I.; Vaidyanathan, P. P.; Herschlag, D. How to measure and evaluate binding affinities. Elife 2020, 9, e57264.].

[Mathematical Formula 10]

$$S = S_{max}(1 - e^{-k_{obs}t})$$

where *t* is the binding time and *S* is the signal of the bound cortisol at that time point. $S_{max}$ reflects the maximum signal of cortisol after equilibrium and $k_{obs}$ expresses the observed rate constant of the binding reaction. $k_{obs}$ is expressed as Mathematical Formula 11:

[Mathematical Formula 11]

$$k_{obs} = k_{on}C + k_{off}$$

where, $k_{on}$ indicates the association rate constant, *C* means the cortisol concentration and $k_{off}$ represents the dissociation rate constant. The association constant ($k_a$) was calculated using the obtained rate constants as Mathematical Formula 12:

[Mathematical Formula 12]

$$k_a = \frac{k_{on}}{k_{off}}$$

**Reference Example 5: Estimation of MICP binding constants by binding adsorption isotherm**

From the *G* measurement, the association constant (*ka*) for the binding events between the MICP and target cortisol was determined using three different adsorption models, namely Langmuir (Mathematical Formula 13), Freundlich (Mathematical Formula 14), and Langmuir-Freundlich models (Mathematical Formula 15). These three models are mainly used to determine the association constant of MIPs because they can more accurately calculate the affinity distribution of the cavities of MIPs, which can have both homogeneous and heterogeneous characteristics [Umpleby, R. J.; Baxter, S. C.; Chen, Y.; Shah, R. N.; Shimizu, K. D. Characterization of molecularly imprinted polymers with the Langmuir Freundlich isotherm. Analytical Chemistry 2001, 73 (19), 4584-4591.].

[Mathematical Formula 13]

$$RR = \frac{RR_{max}K_L C}{K_L C+1}$$

[Mathematical Formula 14]

$$RR = K_F + C^{\mathrm{n}}$$

[Mathematical Formula 15]

$$RR = \frac{RR_{max}{K_{LF}}^{\mathrm{n}}C^{\mathrm{n}}}{K_{LF}C^{\mathrm{n}}+1}$$

where, *RR* is the relative *G* when each concentration is combined, $RR_{max}$ is the maximum *RR,* C is the concentration of cortisol bound to MICP, n is heterogeneity, $K_{LF}$, $K_L$, $K_F$ are the respective constants of the three models. The Langmuir binding model assumes homogenous binding sites while the Freundlich model considers the heterogeneity of the binding sites by incorporating the parameter n, an index of adsorbent heterogeneity, into the binding affinity calculations. The parameter *n* can have values 0 ~ 1. As *n* approaches 1, the heterogeneity of the system decreases and a homogenous system is realized for *n* = 1. The Langmuir-Freundlich model is the combination of the two models which can be used to realize both homogenous and heterogeneous MIPs at saturation and sub-saturation concentrations. The models were fitted to the experimental adsorption isotherms by adjusting three different parameters.

The resulting parameter *K* ($K_{LF}$ for Langmuir-Freundlich model, $K_L$ for Langmuir model) is related to the mean association constant $K_a$.

**Example 1. Electrochemical preparation of MICP**

The bare GCE was polished with 1.0 μm and 0.05 μm alumina powder on a polishingcloth. Then, it was ultrasonically treated at 40 Hz and 70 W in a solution in which ethanol and deionized (DI) water were mixed at a volume ratio of 1:1 for 10 min and an electrochemical potential sweep was carried out in the potential range of -0.2 V to 1.6 V at 100 mV/s (20 scans) in a 0.5 M $H_2SO_4$ solution. Subsequently, the MICP was electrochemically copolymerized in a solution containing 1 mM MB, 100 μM β-CD, and 100 μM cortisol by 10 CV cycles between -2.0 and 2.2 V at a scan rate of 100 mV/s. The extraction of the cortisol template molecule was carried out electrochemically in 1× PBS by CV scanning between 0 and 0.9 V for 10 cycles at a scan rate of 100 mV/s until all cortisol molecules were removed from

the imprinted film.

As a control experiment, the CDP only, PMB only, and NICP were synthesized using the same procedure without MB, β-CD, and the cortisol template, respectively.

The CDP, PMB, and MICP were synthesized on indium tin oxide (ITO) glass (Omniscience, Korea) (for UV-vis) and on the glassy carbon plate (Dasom RMS, Korea) (for SEM and AFM) using the same procedure.

**Example 2. Electrochemical characterization of MICP sensors in buffer solution**

Unless otherwise stated, all electrochemical characterizations were carried out in 1× PBS. CV was performed in the -0.5 V to 0.5 V potential range with a scan rate of 100 mV/s to characterize the properties of the polymer films and the signal stability of PMB in MICPs. After 10 min incubation in cortisol solutions, various cortisol concentrations were measured to obtain a linear regression equation for each electrochemical reaction. All calibration curves were plotted by using the relative response of each electrochemical signal that was obtained by the following Mathematical Formula 16.

[Mathematical Formula 16]

$$Relative\ response\ (RR) = \frac{(R_{[target]} - R_{blank})}{R_{blank}} \times 100$$

where $R_{[target]}$ is the signal transduced for a certain target concentration, and $R_{blank}$ is the signal of the blank. The limit of detection (LOD) of the assay was defined by Mathematical Formula 17.

[Mathematical Formula 17]

$$LOD = 3\frac{\sigma}{slope}$$

where σ is the standard deviation of the standard plot (RR (%) versus target concentration). The quantum EIS was performed between 10 kHz and 0.01 Hz (10 frequencies logarithmically arranged) with a formal DC potential of the PMB (-0.235 V) and a sinusoidal AC perturbation of 20 mV to measure quantum properties at the nanoscale interface. The DPV was performed between 0 V and - 0.4 V, with a pulse amplitude of 50 mV to measure the reduction peak current of PMB. The traditional $R_{ct}$ EIS using an external redox probe was performed in the frequency range of 100 kHz to 0.01 Hz at an applied potential of 0.2 V using an AC voltage of 5.0 mV in a 5.0 mM $K_3Fe(CN)_6/K_4Fe(CN)_6$ solution. Software program CS Studio 5 (Corrtest Instruments Co., Ltd.) has been used to fit the experimental results into an equivalent circuit and estimate the diameter of the semicircles in impedance Nyquist plots and capacitance Nyquist plots. The sensor was regenerated between each measurement by electrochemical extraction as described above (0 to 0.9 V, for 10 cycles). The repeatability was determined by nine reversible measurements of a 100 nM cortisol solution. The cortisol solutions ranging from 1 pM to 1 nM were measured every 2 minutes and plotted as a function of binding time using a one-phase association curve fitting algorithm (see Reference Example 5). The selectivity of cortisol was evaluated by measuring the relative quantum conductance response in the presence of the same concentration of interferents (progesterone and prednisolone) at 10 pM, 1 nM, and 100 nM. In addition, the long-term storage stability was studied for one month by monitoring the relative resonant quantum conductance response for 10 nM cortisol using the MICP-based cortisol sensor, which was kept in a closed and dry chamber at room temperature between each test.

**Example 3. Validation of MICP Sensor Performance with Saliva Sample**

Artificial saliva (AS) was prepared in DI water by adding the major saliva constituents: NaCl (0.4 mg/ml), $CaCl_2$ (0.6 mg/ml), $NaHCO_3$ (0.3 mg/ml), $Na_2HPO_4$ (0.6 mg/ml), KCl (0.4 mg/ml), and urea (4 mg/ml). Afterward, NaOH was added to the solution to obtain a pH of 7.4. The prepared AS was stored at 4 °C until further use. Various cortisol concentrations, within saliva, were measured using in AS for a calibration curve, using the same procedure with the buffer solution. For salivary cortisol monitoring, saliva samples were collected every six hours of the day (8 a.m., 2 p.m., 8 p.m., and 2 a.m.) with commercially available Salivette® (Sarstedt, Nümbrecht, Germany). The Salivette® tubes were centrifuged for 2 min at 1,000×g at 4 °C to remove debris and the supernatant was frozen at -20 °C. The MICP-based

cortisol sensor was repetitively used four times to verify its accuracy and reusability by measuring saliva samples collected at 8 a.m. and 2 a.m., respectively, involving regeneration between each measurement. The circadian rhythms were tracked throughout the day, and the results were validated by LC-MS/MS analysis using Ultimate 3000 RS-Q-Exactive Orbitrap Plus (Thermo Fisher Scientific). Solvent composition and tracking rate parameters for LC were detailed in Table 1 and MS/MS parameters for gas, temperature, and voltage settings were summarized in Table 2.

[Table 1]

LC conditions in positive mode

| No | Time | Flow [ml/min] | % CAN | Curve |
|---|---|---|---|---|
| 1 | 0.000 | Run | | |
| 2 | 0.000 | 0.400 | 5.0 | 5 |
| 3 | 2.000 | 0.400 | 5.0 | 5 |
| 4 | 10.000 | 0.400 | 100.0 | 5 |
| 5 | 14.000 | 0.400 | 100.0 | 5 |
| 6 | 14.500 | 0.400 | 5.0 | 5 |
| 7 | 18.000 | 0.400 | 5.0 | 5 |
| 8 | 18.000 | Stop Run | | |

[Table 2]

MS conditions in positive mode.

| Scan type | Full MS (150-1000 m/z) |
|---|---|
| Polarity | Positive |
| Resolution | 70,000 |
| Sheath gas flow rate | 50 |
| Aux gas flow rate | 13 |
| Spray voltage (kV) | 3.5 |
| Capillary temp. | 320 °C |
| S-lens RF level | 50 |

**[Results and Discussion]**

**1.1 Introducing nanoscale electrochemistry for ultrasensitive detection of small molecules**

The cortisol-selective MICP was prepared by electrochemically copolymerizing β-CD and MB, which resulted in a copolymer film of CDP and PMB. The CDP immobilized within the MICP serves as a recognition element for cortisol and the co-immobilized PMB serves as a redox probe, as shown in FIG. 1A. The thickness of the MICP film was adjusted to the nanoscale level (< 5 nm) in order to increase the target-to-receptor size ratio for efficient signal transduction in the binding events between small cortisol molecules and the artificial CDP receptors. Since conventional $R_{ct}$ EIS is not suitable for the detection of small molecules in the MIP film at the nanoscale level, the inventors of the present invention attempted to analyze the change in capacitance (charge and/or dielectric properties) and other electrochemical properties at the MIP interface. The conventional MIP-based electrochemical sensors operate according to classical mechanics. However, the nanoscale MICP with mesoscopic properties follows quantum mechanics as well as classical mechanics. In the present invention, quantum EIS was adopted to measure the quantum properties of PMB in the MICP under a simultaneous direct current (DC) and alternating current (AC) biases, similar to the conventional $R_{ct}$ EIS method. The PMB acts as a "wire" in which a polymer chain connects the redox site with a resonance charge to the electrode. The binding of cortisol molecules to the cavities of the MICP disrupts the electron transfer through adjacent PMB, which cause the PMB wires to be less conductive, thus leading to a decrease in resonant quantum conductance (see Reference Example 1).

Such phenomenon can also be explained using the equivalent circuit of the MICP (FIG. 1B). In the equivalent circuit, the quantum conductance of the MICP is a constant, which is quantized as $G_0$ ($e^2/h$) in a single electron channel of PMB. The quantum conductance of the MICP can be expressed by Mathematical Formula 18 (Landauer formula):

[Mathematical Formula 18]

$$G = G_0 \sum_n T_n(\mu) = NG_0$$

where $N$ is the number of single electron channels and $\Sigma_n T_n(\mu)$ means the overall transmission probability across the molecular bridge, which is equal to $N$ if the transmittance of each single electron channel is ideal. It implies that the binding of cortisol molecules to the cavities of MICP causes some of the electron channels blocked in an equivalent circuit, thus reducing the number of active electron channels.

Consequently, the binding of cortisol to the MICP induces a decrease in $N$ which in turn leads to a decrease in the conductance of the MICP as explained above. As shown in FIG. 1C, the formal potential ($V_{read}$), set as a DC bias for quantum EIS, is defined as Mathematical Formula 19:

[Mathematical Formula 19]

$$V_{read} = \frac{(V_{red}+V_{ox})}{2}$$

where $V_{read}$ is the electrode overpotential corresponding to the mean energy state between fully reduced and oxidized states of PMB. The fermi energy level with an oxidation and reduction probability of 50%, respectively, is defined as Mathematical Formula 20:

[Mathematical Formula 20]

$$E_F = e\frac{(V_{red}+V_{ox})}{2}$$

where e is the electron charge. Therefore, in the present invention, a DC bias was set to induce the exchange of electrons in the redox states of PMB in order to measure the resonant quantum conductance across the "PMB wire" by applying

a formal potential. When an AC frequency inducing the maximum density of state (DOS) of the film is superimposed on the DC bias, the resonant quantum conductance of the MICP can be calculated by using the electrochemical capacitance ($C_\mu$), solution resistance ($R_s$), and RC time constant term ($\tau$), which are obtained from impedance data obtained from quantum EIS. (As an explanation of quantum electrochemical impedance spectroscopy, data on the impedance of a prepared MIP may be obtained by simultaneously applying a DC voltage corresponding to the median of the electrochemical oxidation/reduction of PMB and an AC voltage capable of repeating oxidation/reduction, and by converting the data using the mathematical formula below, the data as shown in FIG. 6 may be obtained, and the resonant quantum conductance of the MICP may be calculated using the electrochemical capacitance, solution resistance, and RC time constant term obtained through this.) The conversion of impedance data to capacitance data is described in Reference Example 2.

**1.2. Preparation and characterization of nanoscale MICP**

FIG. 2A shows the electrochemical copolymerization process of an MICP utilizing a prepolymer complex consists of β-CD, MB, and cortisol. The polymerization was carried out by applying 10 consecutive potential cycles between -2.0 V and 2.2 V via a CV mode.

The experimental details are described in the Examples.

The first anodic peak at -0.1 V indicates the oxidation of the MB monomer involving the formation of cations on one of the two tertiary amine groups, which further reacts with another tertiary amine group and results in polymerized MB (FIG. 2B). The anodic peak gradually decreases and then disappears at the $6^{th}$ CV cycle. Then, a new anodic peak at 0.45 V begins to increase and becomes saturated from the $8^{th}$ CV cycle. This result indicates that the electrochemical polymerization of PMB was successful.

Simultaneously, β-CD also underwent the polymerization process and showed a well- defined cathodic peak at -0.6 V. From the 2nd CV cycle, additional anodic peaks started to appear at 1.19 V and 1.46 V, which are related to the oxidation of a hydroxyl carbon of β-CD and its further oxidation to carboxylic acid. Subsequently, esterification occurs between the carboxylic and primary hydroxyl groups, forming a dimer, and ultimately resulting in CDP (FIG. 2C).

The anodic peaks were dramatically increased until the 7th CV cycle and started to become saturated in the following cycle. A non-imprinted copolymer (NICP) as a control was also electrochemically copolymerized using the same process as the MICP without the presence of cortisol. CV curves and field emission scanning electron microscope (FE-SEM) images for each polymer film (PMB, CDP, and MICP) were analyzed to confirm the preparation of MICP on a glassy carbon electrode (GCE) surface (FIG. 2D and FIG. 3, respectively). The CV of the MICP film (orange) exhibited clear redox peaks and the CV of PMB-only film without CDP exhibited the same redox peaks, indicating that the PMB is well immobilized in the MICP film. The MICP film showed smaller redox peaks of PMB and a lower double-layer charging capacitance compared to those obtained from the PMB-only film, which is most likely to be due to the incorporation of β-CD in the MICP film. In addition, the peak separation between oxidation and reduction in the MICP film was smaller than that in the PMB-only film. This result can be attributed to the more compact formation of the PMB film, which was facilitated by the established interactions between β-CD and MB. Consequently, the distance between the PMBs was reduced as a result of these interactions.

FE-SEM images obtained for PMB, CDP, and MICP films also confirmed the successful electropolymerization of the proposed MICP film, and the FE-SEM image of the MICP film displayed a more entangled and less porous surface compared to the PMB-only film. Electrochemical extraction of cortisol from the MICP film caused the film more porous, while the electrochemical extraction of cortisol from the control NICP film did not cause a dramatic change in film porosity. This result clearly implies that the morphology change in the MICP film is due to the generation of the receptor cavity within the MICP film, not due to the electrochemical damage of the film. The generation of the receptor cavity in the MICP film was also verified with DPV. The DPV curves obtained from the MICP film showed an increased peak current of PMB oxidation after the extraction of cortisol, which was blocking the current through the PMB, from the MICP film (FIG. 4). The UV-vis spectrum of each polymer film on indium tin oxide (ITO) glass (FIG. 5) displayed two absorption peaks at 611 nm and 662 nm corresponding to PMB for the PMB-only (red) film and MICP film (blue), respectively. But, no absorbance was observed in the CDP-only film.

The MICP also showed a stable redox reaction throughout 40 repeated CV scans (FIG. 2E). The thickness of the MICP film measured by the atomic force microscopy (AFM) image was 4.01 (± 0.97) nm, while the film thickness of CDP and PMB estimated from the surface coverage in their CV curves was 3.11 nm, respectively, (see FIG. 2F and Reference Example 3). The results strongly support that a nanoscale MICP was successfully prepared on a GCE surface, which is expected to have mesoscopic properties.

**1.3. Ultrasensitive detection of cortisol using MICP via quantum EIS**

Traditional $R_{ct}$ EIS, which uses the charge transfer between the electrode and diffusing redox probe in solution,

cannot show the quantum resonant conductance because its measurement needs the DOS exchanging, generated from the charge/discharge of the redox probe anchored to an electrode surface, not from the redox probe in solution.

As described in 1.1 above, quantum EIS using an immobilized PMB redox probe on the GCE surface was performed to investigate quantum electrochemical properties at the interface of the nanoscale MICP film, such as electrochemical capacitance ($C_\mu$), the RC time constant term ($\tau$), and PMB charging resistance ($R_q$). The impedance Nyquist plot shows a decrease in the imaginary impedance component ($Z_{im}$), and the capacitance part of the impedance can be measured when the target cortisol binds to the receptor of the MICP film (FIG. 6A). As shown in FIG. 6B, the relative $Z_{im}$ obtained at 0.08224 Hz was linearly proportional to cortisol in the concentration range from $1.0\times10^{-12}$ M to $1.0\times10^{-6}$ M with a regression equation expressed as follows: relative $Z_{im}$ = 7.58 ($\pm$0.336) log [cortisol] + 17.68 ($\pm$1.13) ($r^2$ = 0.988, n = 4). The limit of detection (LOD) for cortisol was calculated to be $7.01\times10^{-13}$ M (S/N = 3).

The impedance plot was converted to capacitive plots (FIGS. 6C, 6D, and 6E) to provide other quantum properties related to capacitance for more sensitive detection. The diameter of the semicircle in the capacitive Nyquist plot (FIG. 6C) represents $C_\mu$, which can also be attained by the real component of the complex capacitance in the Bode plot at ~ 0.08224 Hz (FIG. 6D), the same frequency used for measuring $Z_{im}$. This $C_\mu$ is related to the charging and discharging characteristics of the PMB redox probes in the MICP film which are interrupted by the binding of cortisol to the receptor-MICP film interface.

Thus, $C_\mu$ decreases as the cortisol concentration increases. As shown in FIG. 6F, the relative $C_\mu$ was linearly proportional to cortisol in the concentration range from $1.0\times10^{-12}$ M to $1.0\times10^{-6}$ M with a regression equation expressed as follows: relative $C_\mu$ = 3.50 ($\pm$0.0748) log [cortisol] + 14.60 ($\pm$0.312) ($r^2$ = 0.997, n = 4). The limit of detection (LOD) for cortisol was calculated to be $5.71\times10^{-13}$ M (S/N = 3).

The $\tau$ is inversely proportional to the peak frequency ($f_p$) of the imaginary capacitive Bode plot (FIG. 6E). The resonant quantum conductance ($G$), which is a reciprocal of $R_q$, was determined from the $f_p$ of the imaginary capacitance Bode plot using the following Mathematical Formula 21:

[Mathematical Formula 21]

$$\tau^{-1} = \frac{1}{(R_s+R_q)C_\mu} = 2\pi f_p$$

The $R_s$ can be obtained from the impedance Nyquist plot at high frequencies as shown in FIG. 6A. The calculated $G$, reflecting the current flow across the PMB "wire" in the MICP film, decreased upon cortisol binding (FIG. 7).

As shown in FIG. 6G, the relative $G$ was linearly proportional to cortisol in the concentration range from $1.0\times10^{-12}$ M to $1.0\times10^{-6}$ M with a regression equation expressed as follows: relative $G$ = 4.34 ($\pm$0.148) log [cortisol] + 25.43 ($\pm$0.658) ($r^2$ = 0.997, n = 4). The limit of detection (LOD) for cortisol was calculated to be $3.93\times10^{-13}$ M (S/N=3).

In addition, the "PMB wire" embedded in the MICP film can be represented by an RC circuit and the electron transfer rate (k) could be represented by the following Mathematical Formula 22:

[Mathematical Formula 22]

$$k = \frac{G}{C_\mu}$$

The electron transfer rate across the "PMB wire" in the MICP film decreased when the target cortisol blocked the electron channel of the "PMB wire" when the binding event occurred (FIG. 6H).

Accordingly, it is certain that the change of $G$ is affected by electron transfer rate as well as capacitance, unlike other quantum properties. These quantum EIS results clearly show that the proposed quantum conductance measurement offers higher sensitivity with a lower LOD for the determination of cortisol compared to the measurements based on other quantum electrochemical responses such as capacitance and electron transfer rate (FIG. 6I).

The cortisol sensing performance of the proposed quantum EIS method in the MICP sensor of the present invention was compared to those obtained from other electrochemical techniques.

First, $R_{ct}$ EIS, utilizing an external redox probe ($Fe(CN)_6^{-3}$/$Fe(CN)_6^{-4}$) solution, was implemented to measure $R_{ct}$ upon binding of cortisol at the nanoscale MICP interface. The resulting Nyquist plot shows that the nanoscale film has too small resistance to induce a significant change in charge transfer resistance upon cortisol binding (FIG. 8).

Instead, when a thickened MICP (electrochemically copolymerized for 40 cycles) was used, $R_{ct}$ increased as the cortisol concentration elevated (FIG. 9). The relative $R_{ct}$ was linearly proportional to cortisol in the much higher concentration range from $1.0\times10^{-8}$ M to $1.0\times10^{-4}$ M with an LOD of $1.59\times10^{-9}$ M (S/N = 3). In addition, DPV was also employed to detect cortisol in the cortisol sensor.

As shown in FIG. 10, the reduction current of PMB in the MICP film decreased as the cortisol concentration increased. The relative reduction current was linearly proportional to cortisol over a narrow concentration range from $1.0\times10^{-10}$ M to $1.0\times10^{-6}$ M with an LOD of $8.49\times10^{-11}$ M (S/N = 3). It is also noteworthy that small molecules can induce noticeable changes in physicochemical properties when bound to receptors within the nanoscale MICP film. While the measurements based on $R_{ct}$ EIS and DPV are not sensitive enough to generate distinct signal changes induced from local changes in response to cortisol binding events because they basically rely on the measurement of redox reactions governed by electron transfer throughout the entire film, the measurement of *G* offers superior sensing performance in terms of sensitivity, detection limit, and dynamic range compared to other traditional methods (see Table 3), as it reflects the local change in charge density of adjacent "PMB wires" embedded in the nanoscale MICP film. Compared to other cortisol sensors, the present invention shows improved limits of detection and linear range. This suggests that the present invention can exhibit improved sensing performance compared to other existing affinity-based label-free (bio)sensors for small molecules (see Table 4).

[Table 3]

Comparison of quantum electrochemical method and conventional electrochemical methods in terms of detection method, sensitivity detection limit, and dynamic range.

| Quantum electrochemical method | sensitivity | LOD (M) | dynamic range (M) |
|---|---|---|---|
| Imaginary impedance component ($Z_{im}$) measurement | 7.58 | $7.01 \times 10^{-13}$ | $1.0 \times 10^{-12}$ - $1.0 \times 10^{-6}$ |
| Electrochemical capacitance ($C_{\mu}$) measurement | 3.50 | $5.71 \times 10^{-13}$ | $1.0 \times 10^{-12}$ - $1.0 \times 10^{-6}$ |
| **Resonant quantum conductance (*G*) measurement** | **4.34** | **$3.93 \times 10^{-13}$** | **$1.0 \times 10^{-12}$ - $1.0 \times 10^{-6}$** |
| **Traditional electrochemical method** | **sensitivity** | **LOD (M)** | **dynamic range (M)** |
| Electrochemical impedance spectroscopy ($R_{ct}$) | 16.85 | $1.59 \times 10^{-9}$ | $1.0 \times 10^{-8}$ - $1.0 \times 10^{-4}$ |
| Differential pulse voltammetry | 1.01 | $8.49 \times 10^{-11}$ | $1.0 \times 10^{-10}$ - $1.0 \times 10^{-6}$ |

[Table 4]

Comparison of various affinity-based electrochemical biosensors in terms of detection method, sensitivity detection limit, and dynamic range.

| **Recognition element** | **Detection method** | **Linear range (M)** | **Limit of detection (M)** | **Reference** |
|---|---|---|---|---|
| Antibody | DPV | $0.5 \times 10^{-9} - 2 \times 10^{-7}$ | $1.10 \times 10^{-10}$ | 1 |
| Antibody | CV, DPV | $2.76 \times 10^{-12} - 27.6 \times 10^{-6}$ | $8.80 \times 10^{-13}$ | 2 |
| Antibody | EIS | $2.76 \times 10^{-12} - 27.6 \times 10^{-9}$ | $2.40 \times 10^{-12}$ | 3 |
| Antibody | CV, DPV | $1 \times 10^{-10} - 1 \times 10^{-4}$ | $1 \times 10^{-10}$ | 4 |
| Cortisol aptamer | EIS | $2.76 \times 10^{-9} - 7.06 \times 10^{-7}$ | $2.76 \times 10^{-9}$ | 5 |
| Cortisol aptamer | EIS | $27.6 \times 10^{-9} - 3.86 \times 10^{-7}$ | $8.27 \times 10^{-9}$ | 6 |
| Cortisol aptamer | SWV | $1.38 \times 10^{-10} - 2.76 \times 10^{-7}$ | $1.38 \times 10^{-10}$ | 7 |
| Cortisol aptamer | EIS | $5 \times 10^{-10} - 1 \times 10^{-8}$ | $1.30 \times 10^{-10}$ | 8 |
| MIP | DPV | $1 \times 10^{-10} - 1 \times 10^{-6}$ | $9.8 \times 10^{-12}$ | 9 |
| MIP | CV | $13.8 \times 10^{-12} - 1.38 \times 10^{-6}$ | $1.93 \times 10^{-11}$ | 10 |
| MIP | CA | $1 \times 10^{-9} - 1 \times 10^{-5}$ | $2 \times 10^{-10}$ | 11 |
| **MIP** | **Quantum conductance** | $\mathbf{1 \times 10^{-12} - 1 \times 10^{-6}}$ | $\mathbf{3.93 \times 10^{-13}}$ | **This work** |

[References]

(1) Campuzano Ruiz, S.; Pedrero, M.; Torrente-Rodriguez, R. M.; Pingarron, J. M. Affinity-Based Wearable Electrochemical Biosensors: Natural versus Biomimetic Receptors. Analysis & Sensing 2022.

(2) Uzun, L.; Turner, A. P. Molecularly-imprinted polymer sensors: Realising their potential. Biosensors and Bioelectronics 2016, 76, 131-144.

(3) Lach, P.; Cieplak, M.; Noworyta, K. R.; Pieta, P.; Lisowski, W.; Kalecki, J.; Chitta, R.; D'Souza, F.; Kutner, W.; Sharma, P. S. Self-reporting molecularly imprinted polymer with the covalently immobilized ferrocene redox probe for selective electrochemical sensing of p-synephrine. Sensors and Actuators B: Chemical 2021, 344, 130276.

(4) Magudeeswaran, V.; Velayutham, J.; Paramasivam, S. S.; Karuppaiah, G.; Mariappan, S. A.; Manickam, P. Self-Reporting Molecularly Imprinted Polymer-Based Electrochemical Sensors for Structurally Similar Analytes. ECS Transactions 2022, 107 (1), 16673.

(5) Zhang, J.; Yang, L.; Pei, J.; Tian, Y.; Liu, J. A reagentless electrochemical immunosensor for sensitive detection of carcinoembryonic antigen based on the interface with redox probe-modified electron transfer wires and effectively

immobilized antibody. Frontiers in Chemistry 2022, 10.
(6) Banerjee, S.; McCracken, S.; Hossain, M. F.; Slaughter, G. Electrochemical detection of neurotransmitters. Biosensors 2020, 10 (8), 101.
(7) Rather, I. A.; Ali, R. Indicator displacement assays: from concept to recent developments. Organic & Biomolecular Chemistry 2021, 19 (27), 5926-5981.
(8) Ebert, M. H.; Schmidt, D. E.; Thompson, T.; Butler, M. G. Elevated plasma gammaaminobutyric acid (GABA) levels in individuals with either Prader-Wili syndrome or Angelman syndrome. The Journal of Neuropsychiatry and Clinical Neurosciences 1997, 9 (1), 75.
(9) Chauhan, D.; Kumar, R.; Panda, A. K.; Solanki, P. R. An efficient electrochemical biosensor for Vitamin-D3 detection based on aspartic acid functionalized gadolinium oxide nanorods. Journal of Materials Research and Technology 2019, 8 (6), 5490-5503.
(10) Huang, L.; Tian, S.; Zhao, W.; Liu, K.; Guo, J. Electrochemical vitamin sensors: A critical review. Talanta 2021, 222, 121645.
(11) Park, S. Y.; Kim, J.; Yim, G.; Jang, H.; Lee, Y.; Kim, S. M.; Park, C.; Lee, M.-H.; Lee, T. Fabrication of electrochemical biosensor composed of multi-functional DNA/rhodium nanoplate heterolayer for thyroxine detection in clinical sample. Colloids and Surfaces B: Biointerfaces 2020, 195, 111240.

**1.4. Characterization of MICP as an artificial receptor**

Affinity-based sensors utilizing classical bioreceptors, such as aptamers and antibodies, are generally suitable for one-time use and also require multiple washing steps with additional regenerating agents (e.g., NaCl, EDTA, NaOH, and SDS, etc.).

On the other hand, the present MICP-based sensor is designed for on-site electrochemical regeneration in the measurement sample solution(due to the electrochemical properties of the used β-CD), enabling repetitive measurements without a washing step. FIG. 11A shows that conductance is recovered to the initial value before cortisol binding after 10 cycles of CV scans, indicating the bound cortisol is completely dissociated from the MICP interface. β-CD may make a 2:1 stoichiometric complex with the cortisol.

The hydrogen bonds in this complex can be formed between β-CD and the carboxyl group of carbon 3, the hydroxyl group of carbon 11, and the hydroxyl group of carbon 21 in the cortisol molecule (FIG. 11B and Chemical Formula 1). CV operation in a controlled potential range (0 V to 0.9 V) may induce the partial oxidation of CDP, leading to a change in the functional groups.

[Chemical Formula 1]

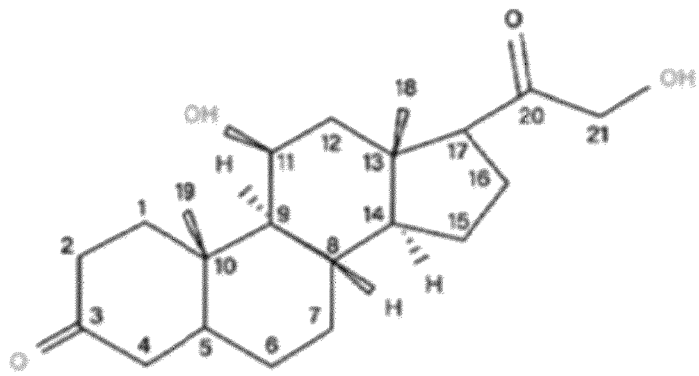

This oxidation can not only generate a radical cation on the carbon but also induces its polarity. The radical cation transforms to a stable resonance form (oxonium ion) and this structure changes may weaken the hydrogen bond, thus cortisol to be extracted from the complex.

This allows MICP-based cortisol sensors to be regenerated up to nine times without any significant loss of sensing performance with a relative standard deviation of 2.05 % (FIG. 11C).

The binding kinetics was studied by measuring different concentrations of cortisol to estimate the association constant ($k_a$) of the MICP (FIG. 11D). The observed rate constants ($k_{obs}$) were plotted against the dedicated cortisol concentration to calculate the parameters related to the binding kinetics (FIG. 11E) and $k_a$ was calculated to be 4.09 ($\pm$ 0.195)$\times 10^{10}$ $M^{-1}$ (see Reference Example 4). The $k_a$ was further evaluated by adsorption isotherms such as Langmuir, Freundlich, and Langmuir-Freundlich models (see Reference Example 5). The fitting parameters and $k_a$ are summarized

in Table 5. The best fitting model was Langmuir-Freundlich ($R^2$ = 0.999), resulting in a $k_a$ of (4.57 ± 2.36)×$10^{10}$ $M^{-1}$ (FIG. 11F). This association constant is much higher than those previously reported for traditional receptors (Table 6). Although a high $k_a$ is essential for ultrasensitive detection, it generally hinders regeneration in traditional bioreceptors.

[Table 5]

Isotherm fitting parameters of each adsorption isotherm model.

| Isotherm Type | Isotherm fitting parameters | | | $R^2$ |
|---|---|---|---|---|
| Langmuir | $RC_{max}$ 41.94 ± 2.88 | $K_L$ (1.07 ± 0.69) x $10^{12}$ | - | 0.830 |
| Freundlich | - | $K_F$ 26.83 ± 0.95 | n 0.05 ± 0.004 | 0.990 |
| Langmuir-Freundlich | $RC_{max}$ 62.31 ± 2.13 | $K_{LF}$ (4.57 ± 2.36) x $10^{10}$ | n 0.14 ± 0.009 | 0.999 |

[Table 6]

Comparison with association constants of conventional bioreceptors.

| Recognition element | $K_a$ ($M^{-1}$) | Reference |
|---|---|---|
| Aptamer | 2.00 x $10^6$ | 12 |
| Aptamer | 3.33 x $10^7$ | 13 |
| Antibody | 2.13 x $10^9$ | 14 |
| MIP | (4.57 ± 2.36) x $10^{10}$ | This study |

[References]

(12) Garrote, B. L.; Santos, A.; Bueno, P. R. Perspectives on and precautions for the uses of electric spectroscopic methods in label-free biosensing applications. ACS Sensors 2019, 4 (9), 2216-2227.

(13) Zamfir, L.-G.; Puiu, M.; Bala, C. Advances in electrochemical impedance spectroscopy detection of endocrine disruptors. Sensors 2020, 20 (22), 6443.

(14) Castro, A. C.; Bezerra, Í R.; Pascon, A. M.; da Silva, G. H.; Philot, E. A.; de Oliveira, V. L.; Mancini, R. S.; Schleder, G. R.; Castro, C. E.; de Carvalho, L. R. Modular labelfree Electrochemical Biosensor Loading Nature-Inspired peptide toward the widespread use of COVID-19 antibody tests. ACS Nano 2022, 16 (9), 14239-14253.

Therefore, although the development of reusable and ultrasensitive affinity-based (bio)sensors is challenging the MICP manufactured in the present invention not only is more advantageous for ultrasensitive detection due to its high association constant, but also can be regenerated by electrochemically-triggered structural changes without multiple washing steps or regenerating reagents. Steroids are chemical compounds with a core structure composed of four

"fused" rings and only vary by the functional groups attached to the core rings. This causes their hard to distinguish the specific steroid from others, requiring a high degree of selectivity for steroid (bio)sensors. The relative conductance measured by MICP and NICP in the presence of the same concentration of cortisol and structural analogs (progesterone and prednisolone) showed excellent selectivity for these interferents in MICP (FIG. 11G and FIG. 12). Considering the proposed interaction between cortisol and CDP, prednisolone has similar functional groups to cortisol, compared to progesterone, which resulted in slightly higher interference. In addition, the MICP showed excellent storage stability at room temperature during the 4-week storage period (FIG. 11H).

**1.5. Validation of MICP cortisol sensor through saliva samples**

Cortisol is a steroid hormone secreted in response to stress, and thus plays an important role as a stress biomarker.

Chronic stress is associated with increased risk of mental health, weakening immune responses, and cardiovascular disease. Therefore, effective and reliable cortisol detection is very useful for monitoring the fluctuations of cortisol throughout the day toward comprehensive self-monitoring and personalized healthcare.

Salivary cortisol, which can be easily collected non-invasively, is known to be highly correlated with blood cortisol levels.

Cortisol levels vary according to the circadian rhythm, with concentrations peaking 30 min after waking up and lowest around bedtime.

The present MICP-based cortisol sensor was evaluated in artificial saliva (AS) using quantum EIS. The resulting capacitance Bode plots are shown in FIGS. 13A and 13B. These Bode plots display $C_\mu$ and $f_p$ at the same frequency (0.08224 Hz and 1.35542 Hz, respectively) as in 1x PBS, due to the similarity of the solution medium. A plot of relative conductance shows a linear regression equation as $RR$ = 4.21 ($\pm$0.148) log [cortisol] + 26.29 ($\pm$0.658) ($r^2$ = 0.998, n = 4), covering the physiological salivary cortisol level (2.2 ~ 27.3 nM) (FIG. 13C).

The accuracy for the determination of saliva based on the present MICP-based sensor was validated by LC-MS/MS analysis (FIG. BD). Repetitive measurement (n = 4) of saliva samples collected at 2 a.m. and 8 a.m., including electrochemical regeneration steps, shows a relative quantum conductance of 39.69 ($\pm$0.039)% and 40.88 ($\pm$0.044)%, respectively, demonstrating the proposed cortisol sensor can generate consistent sensing signals and regeneration in human saliva (FIG. 13E). As a result of further evaluating the circadian rhythm of the salivary cortisol level throughout the day, the highest value was shown in the morning (8 a.m.) and gradually decreased until the evening (2 a.m.) (FIG. 13F). The measured salivary cortisol levels show a differenceof less than 3% (n = 3) between the developed cortisol sensor and LC-MS/MS (Table 7). These results clearly show that the proposed cortisol sensing system can be used for practical "continuous bind-and-read" salivary stress monitoring.

[Table 7]

Comparison of saliva cortisol measured using LC-MS/MS and MICP cortisol sensor.

| Time of saliva samples obtained | Salivary cortisol (LC-MS/MS) (nM) | RSD (LC-MS/MS) (n=3) (%) | Salivary cortisol (MICP) (nM) | RSD (MICP) (n=3) (%) | Difference between LC-MS/MS and MICP (%) |
|---|---|---|---|---|---|
| 8 a.m. | 2.88 | 2.83 | 2.96 | 5.79 | 2.83 |
| 2 p.m. | 2.23 | 1.44 | 2.19 | 2.28 | 1.44 |
| 8 p.m. | 1.74 | 0.88 | 1.72 | 1.74 | 0.88 |
| 2 a.m. | 1.52 | 0.56 | 1.5 | 1.52 | 0.56 |

**[Conclusions]**

In the present invention, quantum electrochemistry was utilized to overcome the existing limitations of affinity-based (bio)sensors for small molecules. Various electrochemical methods have been employed for the manufacture of MICPs, cortisol detection, and sensor regeneration.

Using CV, the nanoscale MICP film with an embedded redox probe can be easily synthesized and easily regenerated without any need of lengthy extraction or several washing steps. The quantum EIS, which measures resonant quantum conductance, enables label-free, ultrasensitive, and selective cortisol monitoring through the decreased conductance response of the "PMB wire" immobilized in the MICP. By integrating mass-producible MICP and the conductance measurement, we have demonstrated an in-situ and continuous cortisol monitoring strategy, promising for wearable health monitoring applications. Using the present MICP-based cortisol sensor, we have successfully measured the salivary cortisol variations following the circadian rhythm and have thoroughly validated using an LC-MS/MS. The nanoscale MICP sensor of the present invention, which is capable of conductance measurements, may be readily expanded to detect various other small molecules. Overall, the new MICP-based cortisol sensing enabling "continuous bind-and-read" provides a reliable and practical approach for in-situ ultrasensitive stress monitoring. The use of quantum electrochemistry with nanoscale MICP for tackling the limitations of affinity-based (bio)sensor paves the way to a new possibility of (bio)sensor applications toward the PoC and wearable device for healthcare..

The sensor according to the present invention can be reused by repeating the oxidation/reduction of a receptor through CV and repeating the separation/binding of the receptor and a small molecule. In other words, when CV is used, nano-sized MIP films with an embedded redox probe can be easily synthesized and easily regenerated without lengthy extraction or multiple washing steps. Quantum EIS for measuring resonant quantum conductance enables label-free, ultrasensitive selective monitoring through the reduced conductance response of a 'redox probe wire' immobilized in the MIP. Integrating conductance measurements with mass-producible MIPs enables on-site and continuous monitoring suitable for wearable health monitoring applications.

**Claims**

1. A nanoscale molecularly imprinted polymer thin film, which is a molecularly imprinted polymer thin film for small molecule detection, in which a plurality of specific recognition spaces for small molecules of 1000 Da or less are formed,

   wherein a receptor polymer is present at one end of the specific recognition spaces, and Prussian blue, ferrocene or polymethylene blue is present in a wire form as a redox probe, and
   the small molecule is a steroid hormone.

2. The molecularly imprinted polymer thin film of claim 1, wherein the molecularly imprinted polymer thin film is manufactured by electrochemically copolymerizing a small molecule, β-cyclodextrin, and methylene blue, and then removing the small molecule.

3. The molecularly imprinted polymer thin film of claim 1, wherein the small molecule is cortisol or melatonin.

4. The molecularly imprinted polymer thin film of claim 1, wherein the nanoscale molecularly imprinted polymer thin film has a thickness of 5 nm or less.

5. The molecularly imprinted polymer thin film of claim 1, wherein the receptor polymer is formed from β-cyclodextrin, pyrrole, or phenylenediamine monomer.

6. A method of manufacturing a nanoscale molecularly imprinted polymer thin film for small molecules, comprising:

   forming a molecularly imprinted polymer thin film for small molecules on an electrode of an electrochemical sensing device; and
   removing the small molecule.

7. The method of claim 6, wherein the electrode is pretreated by ultrasonic treatment in ethanol and deionized water and then performing an electrochemical potential sweep.

8. The method of claim 6, wherein the molecularly imprinted polymer thin film for small molecules is formed by electrochemically copolymerizing a small molecule, β-cyclodextrin, and methylene blue.

9. The method of claim 6, wherein the molecularly imprinted polymer thin film is manufactured by a polymerization

reaction of a small molecule, a receptor polymer, and a redox probe at a molarity(M) ratio of 1:0.5 to 1.5:5 to 15.

**10.** A method of detecting small molecules, comprising:

bringing a biological sample into contact with a quantum electrochemical detection device including the molecularly imprinted polymer thin film of claim 1;
applying voltage to the quantum electrochemical detection device; and
monitoring changes in resonant quantum conductance from the device when a target small molecule binds to a specific recognition space inside the molecularly imprinted polymer thin film.

**11.** The method of claim 10, wherein the biological sample is selected from the group consisting of plasma, serum, saliva, urine, mucus, and tears.

**12.** The method of claim 10, wherein the quantum electrochemical detection device is a device that measures resonant quantum conductance using quantum electrochemical impedance spectroscopy (EIS).

**13.** The method of claim 10, wherein the small molecule is detected at a concentration of $10\times10^{-13}$ to $1.0\times10^{-6}$.

**14.** An electrochemical biosensor for small molecule detection including the molecularly imprinted polymer thin film of claim 1.

Fig. 1

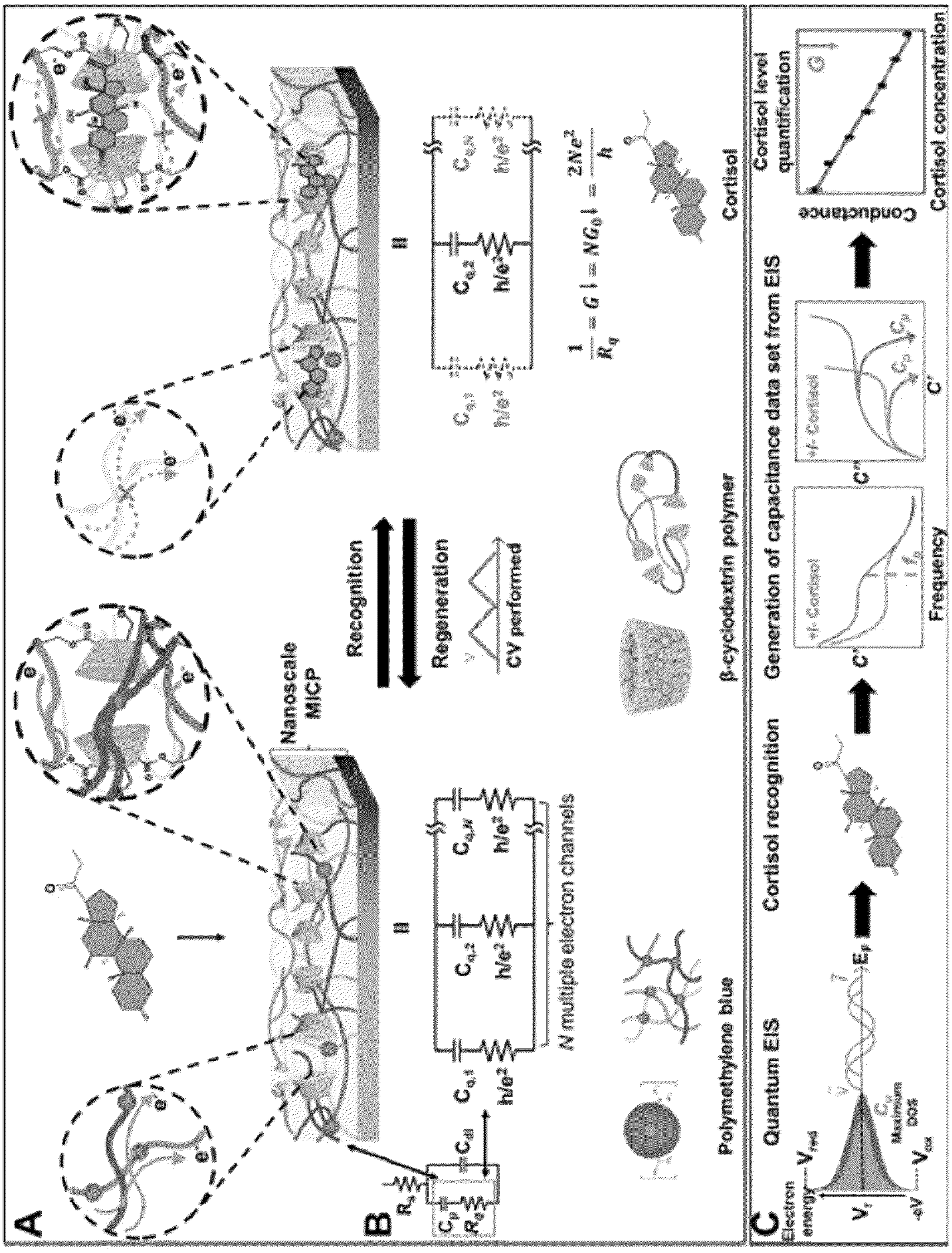
A
B
C
Nanoscale MICP
Recognition
Regeneration
CV performed
N multiple electron channels
Polymethylene blue
β-cyclodextrin polymer
Cortisol
Quantum EIS
Cortisol recognition
Generation of capacitance data set from EIS
Cortisol level quantification
Frequency
Conductance
Cortisol concentration
Electron energy
+/- Cortisol

Fig. 2

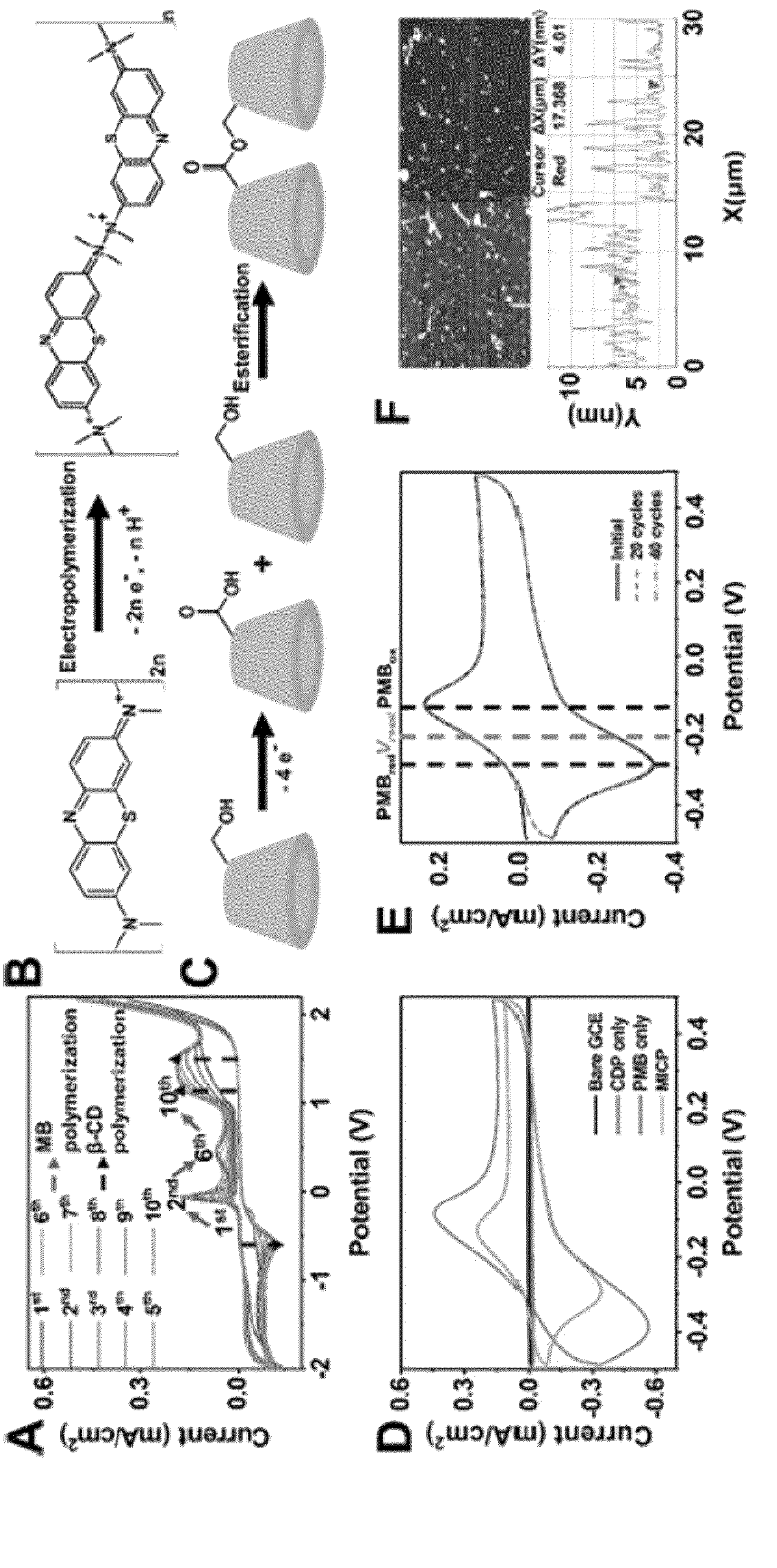
A
B
C
D
E
F
Current (mA/cm²)
Potential (V)
MB polymerization
β-CD polymerization
Electropolymerization
Esterification
Bare GCE
CDP only
PMB only
MICP
Initial
20 cycles
40 cycles
Y(nm)
X(μm)

Fig. 3

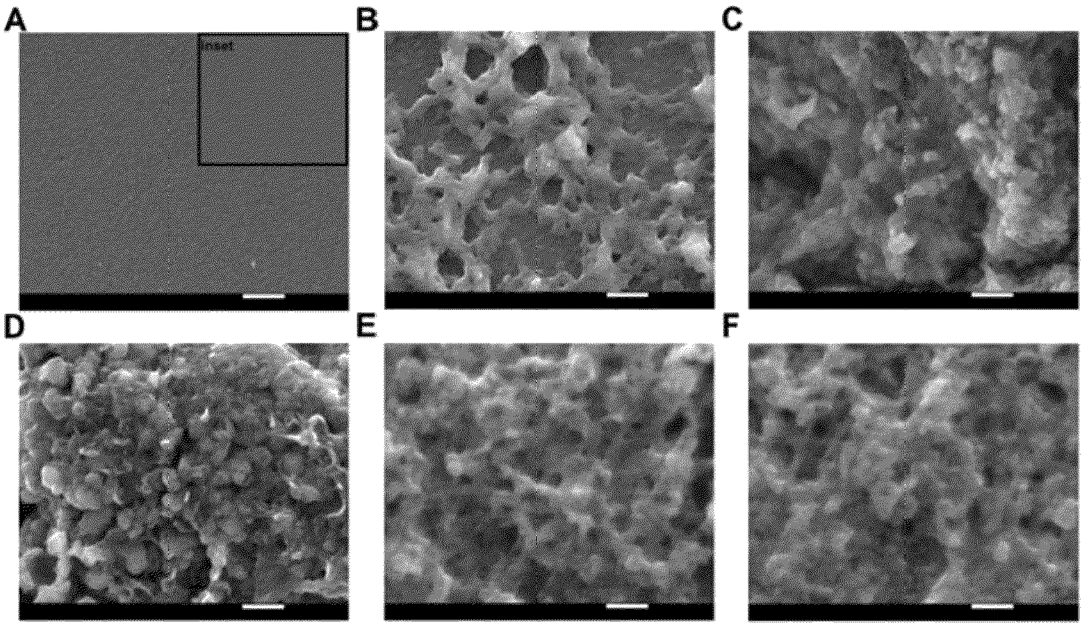

Fig. 4

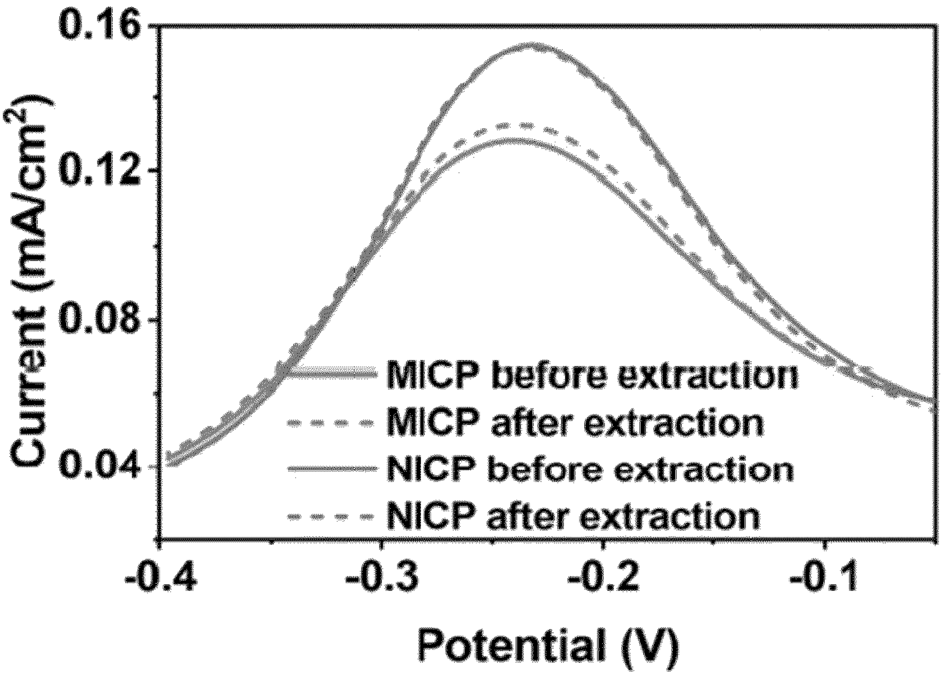
0.16
0.12
0.08
0.04
Current (mA/cm²)
-0.4
-0.3
-0.2
-0.1
Potential (V)
MICP before extraction
MICP after extraction
NICP before extraction
NICP after extraction

Fig. 5

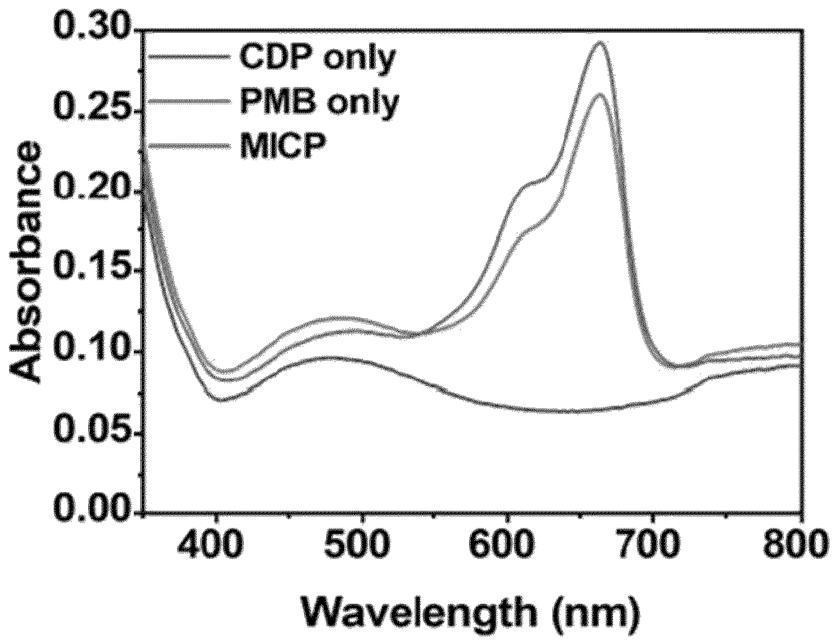
0.30
0.25
0.20
0.15
0.10
0.05
0.00
Absorbance
CDP only
PMB only
MICP
400
500
600
700
800
Wavelength (nm)

Fig. 6

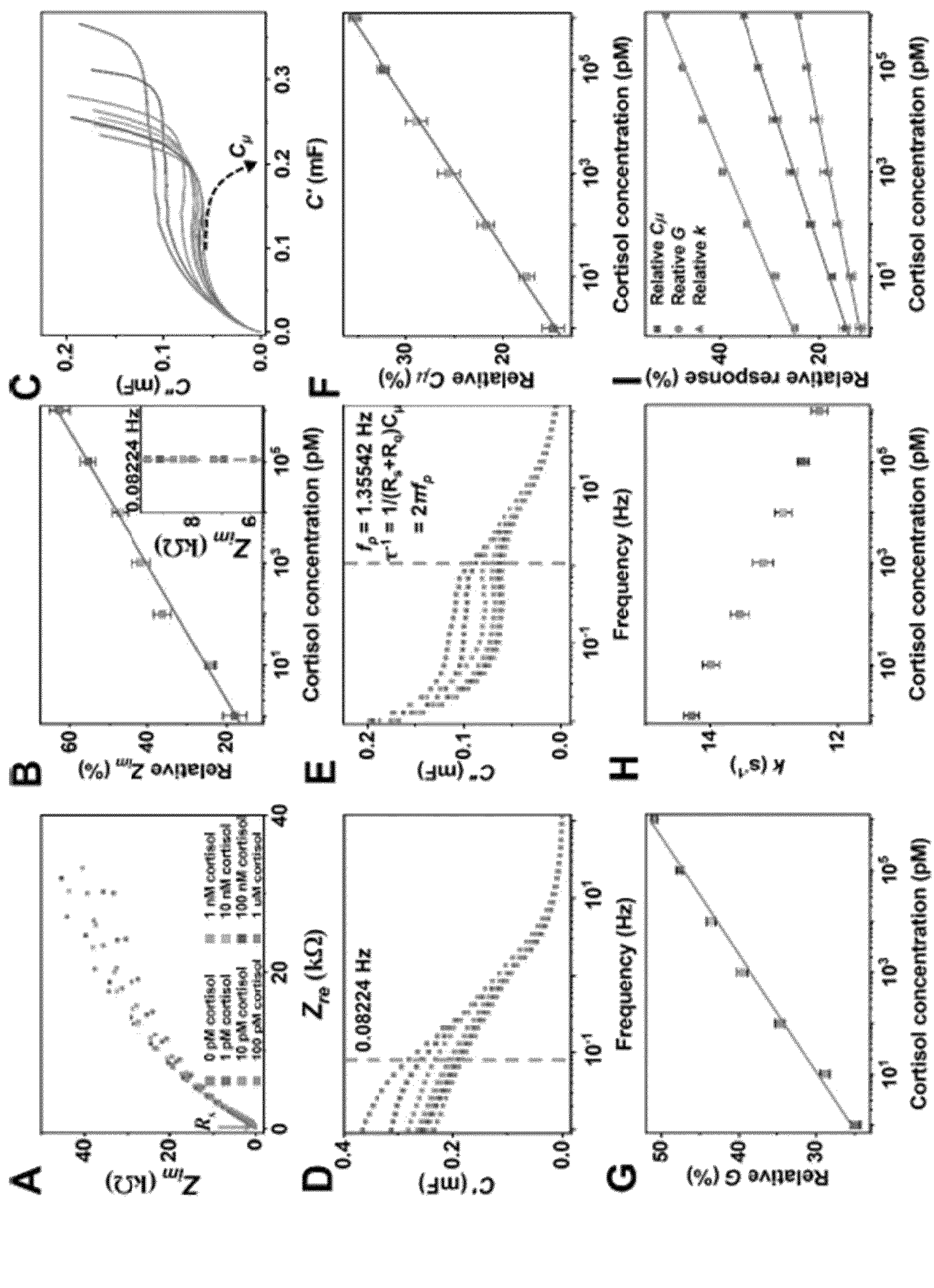
A
B
C
D
E
F
G
H
I
$Z_{im}$ (kΩ)
$Z_{re}$ (kΩ)
0 pM cortisol
1 pM cortisol
10 pM cortisol
100 pM cortisol
1 nM cortisol
10 nM cortisol
100 nM cortisol
1 uM cortisol
Relative $Z_{im}$ (%)
0.08224 Hz
C'' (mF)
C' (mF)
$C_\mu$
Frequency (Hz)
$f_p$ = 1.35542 Hz
$\tau^{-1} = 1/(R_s+R_o)C_\mu$
$= 2\pi f_p$
Relative $C_\mu$ (%)
Relative G (%)
k ($s^{-1}$)
Relative response (%)
Relative $C_\mu$
Relative G
Relative k
Cortisol concentration (pM)

Fig. 7

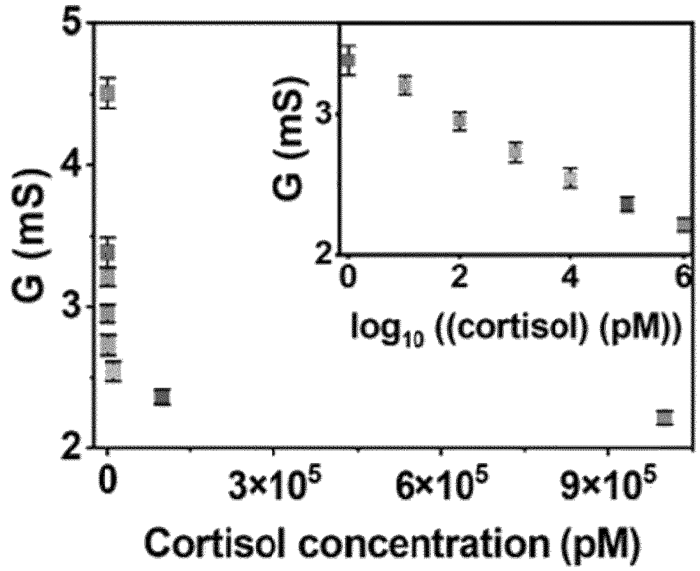

5
4
3
2
G (mS)
0
3×10^5
6×10^5
9×10^5
Cortisol concentration (pM)
G (mS)
3
2
0
2
4
6
log10 ((cortisol) (pM))

Fig. 8

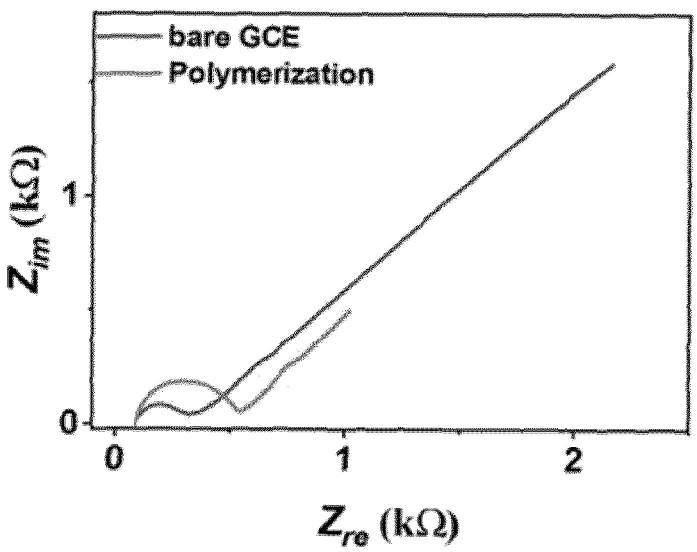

Fig. 9

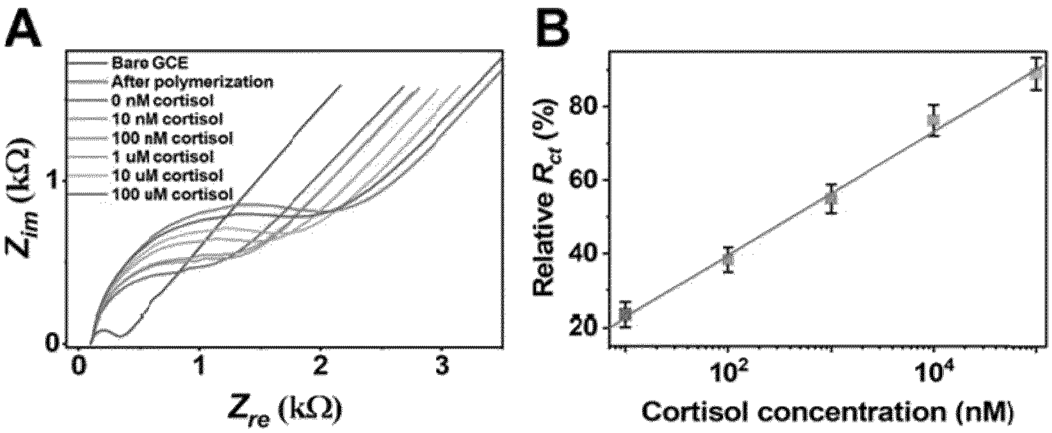
A
Bare GCE
After polymerization
0 nM cortisol
10 nM cortisol
100 nM cortisol
1 uM cortisol
10 uM cortisol
100 uM cortisol
$Z_{im}$ (kΩ)
$Z_{re}$ (kΩ)
B
Relative $R_{ct}$ (%)
Cortisol concentration (nM)

Fig. 10

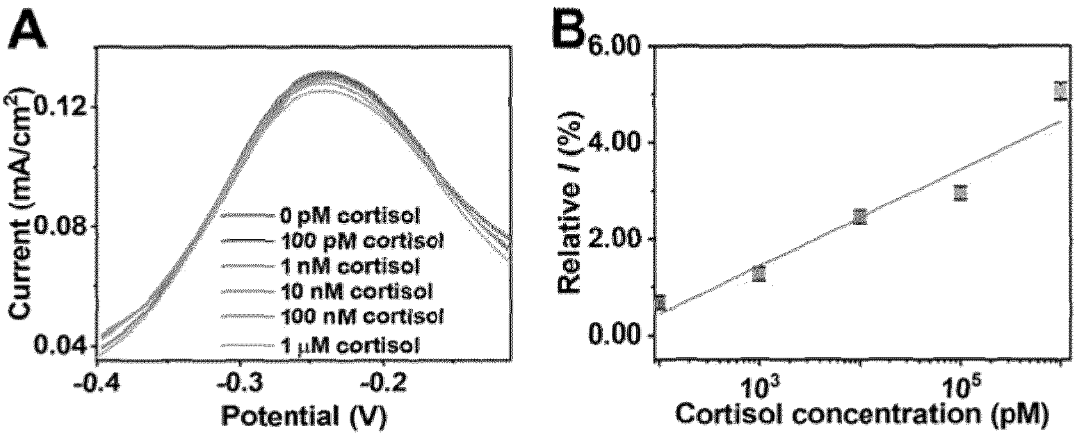
A
Current (mA/cm$^2$)
0.12
0.08
0.04
-0.4
-0.3
-0.2
Potential (V)
0 pM cortisol
100 pM cortisol
1 nM cortisol
10 nM cortisol
100 nM cortisol
1 μM cortisol
B
Relative I (%)
6.00
4.00
2.00
0.00
10$^3$
10$^5$
Cortisol concentration (pM)

Fig. 11

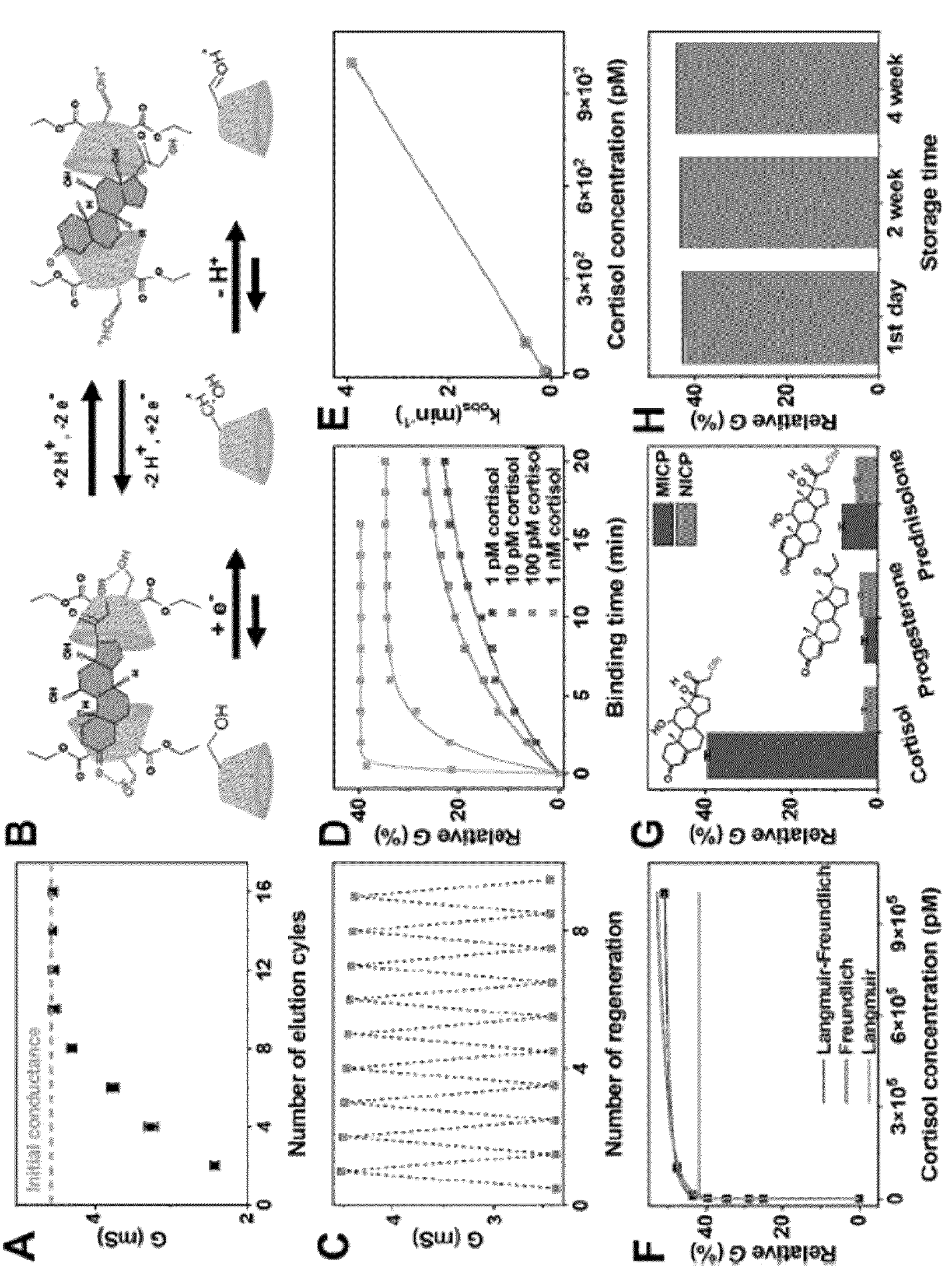
A
Initial conductance
G (mS)
Number of elution cyles
B
+ e-
+2 H+, -2 e-
-2 H+, +2 e-
- H+
C
G (mS)
Number of regeneration
D
Relative G (%)
1 pM cortisol
10 pM cortisol
100 pM cortisol
1 nM cortisol
Binding time (min)
E
kobs(min-1)
Cortisol concentration (pM)
F
Relative G (%)
Langmuir-Freundlich
Freundlich
Langmuir
Cortisol concentration (pM)
G
Relative G (%)
MICP
NICP
Cortisol
Progesterone
Prednisolone
H
Relative G (%)
1st day
2 week
4 week
Storage time

Fig. 12

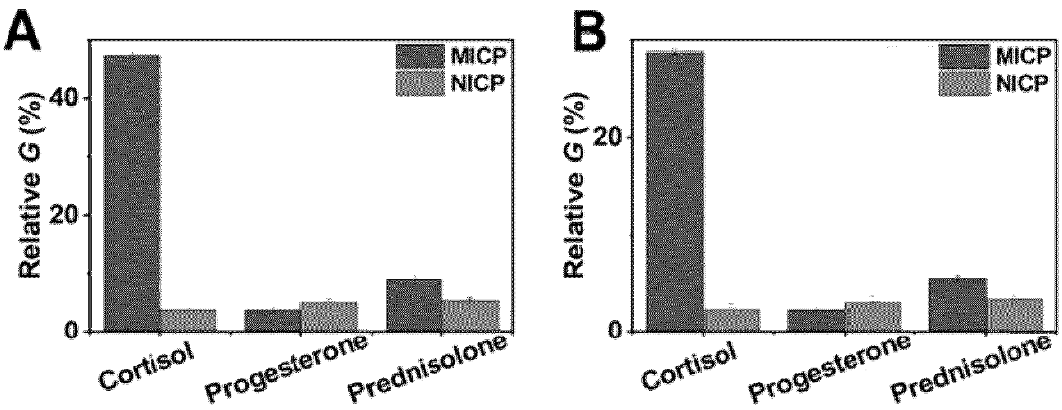
A
B
MICP
NICP
Relative G (%)
0
20
40
Cortisol
Progesterone
Prednisolone

Fig. 13

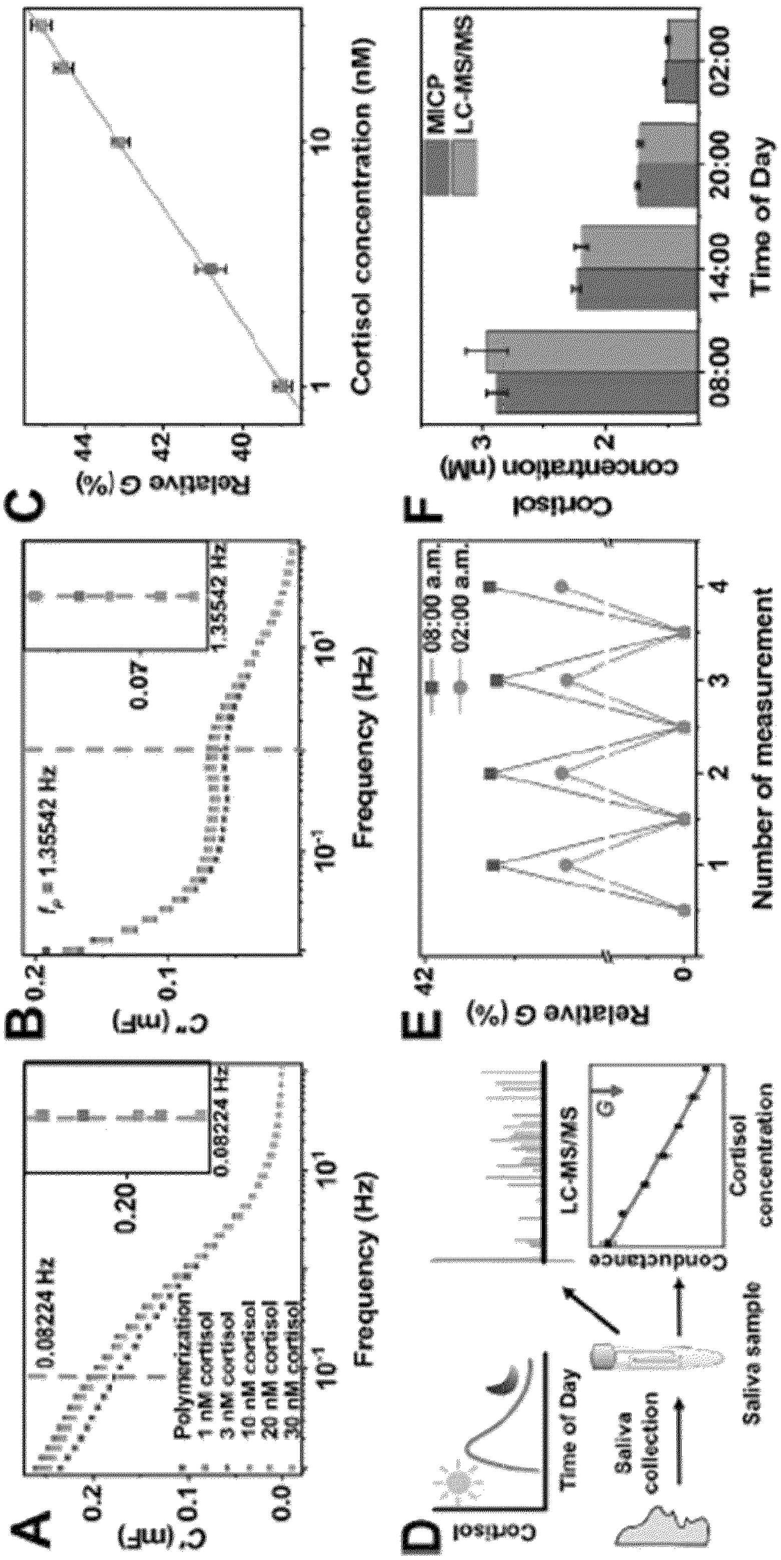
A
C' (mF)
0.08224 Hz
Polymerization
1 nM cortisol
3 nM cortisol
10 nM cortisol
20 nM cortisol
30 nM cortisol
0.20
0.08224 Hz
Frequency (Hz)
B
C" (mF)
$f_p$ = 1.35542 Hz
0.07
1.35542 Hz
Frequency (Hz)
C
Relative G (%)
Cortisol concentration (nM)
D
Cortisol
Time of Day
Saliva collection
Saliva sample
LC-MS/MS
Conductance
Cortisol concentration
E
Relative G (%)
08:00 a.m.
02:00 a.m.
Number of measurement
F
Cortisol concentration (nM)
MICP
LC-MS/MS
08:00
14:00
20:00
02:00
Time of Day

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20230081703


### Non-patent literature cited in the description


- **KIM, Y. ; YANG, J. ; HUR, H. ; OH, S. ; LEE, H. H.** Highly sensitive colorimetric assay of cortisol using cortisol antibody and aptamer sandwich assay. *Biosensors,* 2021, vol. 11 (5), 163
- **DING, L. ; WANG, Z. ; ZHONG, P. ; JIANG, H. ; ZHAO, Z. ; ZHANG, Y. ; REN, Z. ; DING, Y.** Structural insights into the mechanism of single domain VHH antibody binding to cortisol. *FEBS letters,* 2019, vol. 593 (11), 1248-1256
- **APODACA, D. C. ; PERNITES, R. B. ; PONNAPATI, R. ; DEL MUNDO, F. R. ; ADVINCULA, R. C.** Electropolymerized molecularly imprinted polymer film: EIS sensing of bisphenol A. *Macromolecules,* 2011, vol. 44 (17), 6669-6682
- **BUENO, P. R. ; FERNANDES, F. C. B. ; DAVIS, J. J.** Quantum capacitance as a reagentless molecular sensing element. *Nanoscale,* 2017, vol. 9 (40), 15362-15370
- **BUENO, P. R.** Nanoscale Electrochemistry of Molecular Contacts. Springer, 2018
- **GARROTE, B. L. ; SANTOS, A. ; BUENO, P. R.** Label-free capacitive assaying of biomarkers for molecular diagnostics. *Nature Protocols,* 2020, vol. 15 (12), 3879-3893
- **MARINHO, M. I. C. ; CABRAL, M. F. ; MAZO, L. H.** Is the poly (methylene blue)-modified glassy carbon electrode an adequate electrode for the simple detection of thiols and amino acid-based molecules?. *Journal of Electroanalytical Chemistry,* 2012, vol. 685, 8-14
- **TAN, L. ; XIE, Q. ; YAO, S.** Electrochemical and spectroelectrochemical studies on pyridoxine hydrochloride using a poly (methylene blue) modified electrode. *Electroanalysis: An International Journal Devoted to Fundamental and Practical Aspects of Electroanalysis,* 2004, vol. 16 (19), 1592-1597
- **SANDILYA, A. A. ; NATARAJAN, U. ; PRIYA, M. H.** Molecular view into the cyclodextrin cavity: structure and hydration. *ACS Omega,* 2020, vol. 5 (40), 25655-25667
- **ZEILINGER, M. ; PICHLER, F. ; NICS, L. ; WADSAK, W. ; SPREITZER, H. ; HACKER, M. ; MITTERHAUSER, M.** New approaches for the reliable in vitro assessment of binding affinity based on high-resolution real-time data acquisition of radioligand-receptor binding kinetics. *EJNMMI Research,* 2017, vol. 7 (1), 1-13
- **JARMOSKAITE, I. ; ALSADHAN, I. ; VAIDYANATHAN, P. P. ; HERSCHLAG, D.** How to measure and evaluate binding affinities. *Elife,* 2020, vol. 9, e57264
- **UMPLEBY, R. J. ; BAXTER, S. C. ; CHEN, Y. ; SHAH, R. N. ; SHIMIZU, K. D.** Characterization of molecularly imprinted polymers with the Langmuir Freundlich isotherm. *Analytical Chemistry,* 2001, vol. 73 (19), 4584-4591
- **CAMPUZANO RUIZ, S. ; PEDRERO, M. ; TORRENTE-RODRIGUEZ, R. M. ; PINGARRON, J. M.** Affinity-Based Wearable Electrochemical Biosensors: Natural versus Biomimetic Receptors. *Analysis & Sensing,* 2022
- **UZUN, L. ; TURNER, A. P.** Molecularly-imprinted polymer sensors: Realising their potential. *Biosensors and Bioelectronics,* 2016, vol. 76, 131-144
- **LACH, P. ; CIEPLAK, M. ; NOWORYTA, K. R. ; PIETA, P. ; LISOWSKI, W. ; KALECKI, J. ; CHITTA, R. ; D'SOUZA, F. ; KUTNER, W. ; SHARMA, P. S.** Self-reporting molecularly imprinted polymer with the covalently immobilized ferrocene redox probe for selective electrochemical sensing of p-synephrine. *Sensors and Actuators B: Chemical,* 2021, vol. 344, 130276
- **MAGUDEESWARAN, V. ; VELAYUTHAM, J. ; PARAMASIVAM, S. S. ; KARUPPAIAH, G. ; MARIAPPAN, S. A. ; MANICKAM, P.** Self-Reporting Molecularly Imprinted Polymer-Based Electrochemical Sensors for Structurally Similar Analytes. *ECS Transactions,* 2022, vol. 107 (1), 16673

- **ZHANG, J. ; YANG, L. ; PEI, J. ; TIAN, Y. ; LIU, J.** A reagentless electrochemical immunosensor for sensitive detection of carcinoembryonic antigen based on the interface with redox probe-modified electron transfer wires and effectively immobilized antibody. *Frontiers in Chemistry,* 2022, vol. 10
- **BANERJEE, S. ; MCCRACKEN, S. ; HOSSAIN, M. F. ; SLAUGHTER, G.** Electrochemical detection of neurotransmitters. *Biosensors,* 2020, vol. 10 (8), 101
- **RATHER, I. A. ; ALI, R.** Indicator displacement assays: from concept to recent developments. *Organic & Biomolecular Chemistry,* 2021, vol. 19 (27), 5926-5981
- **EBERT, M. H. ; SCHMIDT, D. E. ; THOMPSON, T. ; BUTLER, M. G.** Elevated plasma gammaaminobutyric acid (GABA) levels in individuals with either Prader-Wili syndrome or Angelman syndrome. *The Journal of Neuropsychiatry and Clinical Neurosciences,* 1997, vol. 9 (1), 75
- **CHAUHAN, D. ; KUMAR, R. ; PANDA, A. K. ; SOLANKI, P. R.** An efficient electrochemical biosensor for Vitamin-D3 detection based on aspartic acid functionalized gadolinium oxide nanorods. *Journal of Materials Research and Technology,* 2019, vol. 8 (6), 5490-5503
- **HUANG, L. ; TIAN, S. ; ZHAO, W. ; LIU, K. ; GUO, J.** Electrochemical vitamin sensors: A critical review. *Talanta,* 2021, vol. 222, 121645
- **PARK, S. Y. ; KIM, J. ; YIM, G. ; JANG, H. ; LEE, Y. ; KIM, S. M. ; PARK, C. ; LEE, M.-H. ; LEE, T.** Fabrication of electrochemical biosensor composed of multi-functional DNA/rhodium nanoplate heterolayer for thyroxine detection in clinical sample. *Colloids and Surfaces B: Biointerfaces,* 2020, vol. 195, 111240
- **GARROTE, B. L. ; SANTOS, A. ; BUENO, P. R.** Perspectives on and precautions for the uses of electric spectroscopic methods in label-free biosensing applications. *ACS Sensors,* 2019, vol. 4 (9), 2216-2227
- **ZAMFIR, L.-G. ; PUIU, M. ; BALA, C.** Advances in electrochemical impedance spectroscopy detection of endocrine disruptors. *Sensors,* 2020, vol. 20 (22), 6443